(19)

**Europäisches Patentamt**
**European Patent Office**
**Office européen des brevets**

(11) **EP 4 678 167 A1**

(12) **EUROPEAN PATENT APPLICATION**

(43) Date of publication:
14.01.2026 Bulletin 2026/03

(21) Application number: 25185883.3

(22) Date of filing: 27.06.2025

(51) International Patent Classification (IPC):
$A61K\ 9/20^{(2006.01)}$ $A61K\ 31/05^{(2006.01)}$
$A61P\ 9/12^{(2006.01)}$ $A61P\ 15/12^{(2006.01)}$
$A61P\ 19/10^{(2006.01)}$ $A61P\ 35/00^{(2006.01)}$

(52) Cooperative Patent Classification (CPC):
A61K 9/2063; A61K 31/05; A61P 9/00; A61P 9/12;
A61P 15/12; A61P 19/10; A61P 35/00

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 04.07.2024 IT 202400015469

(71) Applicant: Laboratorio della Farmacia S.p.A.
30020 Quarto d'Altino VE (IT)

(72) Inventors:
• Fratter, Andrea
30020 Quarto d'Altino VE (IT)
• Salvagnin, Massimo
30020 Quarto d'Altino VE (IT)
• Cignarella, Andrea
30020 Quarto d'Altino VE (IT)

(74) Representative: Viganò, Jessica
Studio Legale Viganò
Via Sapeto, 1
20123 Milano (IT)

(54) **SOLID ORAL COMPOSITION WITH GRADUAL ENTERIC RELEASE FOR LIPOPHILIC ACTIVE INGREDIENTS**

(57) The present patent application concerns a gradual enteric-release oral solid composition comprising: i) at least one active lipophilic ingredient belonging to BCS class II and/or IV according to the biopharmaceutical classification system; ii) a self-emulsifying vehicle comprising or consisting of ii-a) a solubilizing oily matrix comprising or consisting of a polyoxyethylene sorbitan ester with a fatty acid, the fatty acid being saturated or unsaturated and having a number of carbon atoms comprised between 16 and 22, a mono and diglyceride of a fatty acid, the fatty acid being saturated or unsaturated and having a number of carbon atoms comprised between 16 and 22, at least one unsaturated fatty acid and having a number of carbon atoms comprised between 16 and 20, ii-b) a caseinate derivative; iii) suitable excipients and/or diluents, wherein the ratio, expressed in formula (I), between the amount of the caseinate derivative and the ratio between the amount of the at least one ester of the polyoxyethylene sorbitan and the amount of the at least one active lipophile is comprised between 0.03 and 0.6.

Disaggregation RES LIBADDS 013 vs. RES LIBADDS 017 vs. RES LIBADDS 019

Figure 13

EP 4 678 167 A1

**Description**

**FIELD OF THE INVENTION**

The present invention falls within the field of technologies that promote the gradual release of an active lipophilic ingredient, poorly soluble in water, in the enteric context starting from a solid composition for oral use, for example from a tablet.

**STATE OF THE ART**

Today nutraceuticals represent a promising strategy for the maintenance of health and a rapidly growing and continuously expanding market in Italy and, in general, in Europe. The lack of strict regulation regarding formulation requirements and efficacy evidence highlights the serious problem of their poor bioavailability.

*Problems of the background art*

The administration of active lipophilic ingredient substances represents a challenge from a technological and pharmacological point of view, given the need to make them dispersible in aqueous-based enteric fluids in order to be absorbed, and possibly to protect them against pre-systemic enteric metabolism.

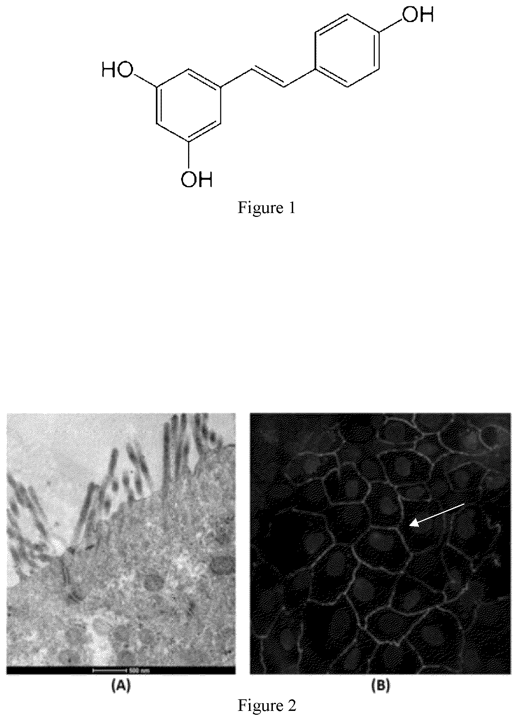

An example of an active lipophilic ingredient, poorly soluble in the aqueous medium, with high intestinal permeability but which undergoes a massive pre-systemic enteric metabolism, is t-resveratrol (RES), a polyphenolic stilbene (Figure 1) extracted from several plants, in particular from the seeds and skin of *Vitis Vinifera* and from the roots of *Polygonum Cuspidatum.* The RES has aroused the interest of pharmacologists and clinicians in the last thirty years, for its proven cardioprotective effects [1-3], for its estrogen-like activity in preventing osteopenia and cognitive decline in menopause [4,5], as well as for its ability to modulate the activity of caspase and sirtuin enzymes [6,7].

Despite good intestinal bioaccessibility, as reported by Walle, the bioavailability of RES is low and limited in humans [8,9], much lower than 1%, since the active in question undergoes extensive metabolism in the intestine and liver. More in detail, RES is massively metabolized in liver microsomes and intestinal cells by phase II enzymes and in particular by UDP-glucuronyl transferase and, consequently, the glucosides and sulfates of RES are the main metabolites present in the blood [10-12].

In this perspective, new approaches aiming at releasing active lipophilic ingredient molecules in emulsified or micellized form are attracting more and more attention and technological efforts. Of these, *Self-Emulsifying Drug Delivery Systems* (SEDDS) and *Lipid-Based Drug Delivery Systems* (LiBADDS) are the most widely described and cited in the literature [13-17]. These delivery systems, in fact, promote the dispersion of lipophilic or poorly soluble active substances in the form of an emulsion ready for micellar dispersion on contact with gastro-enteric fluids.

Known compositions, for example solid compositions that promote the formation of *in vivo* and prolonged release emulsions, provide for the use of adsorbing agents, such as silicates, cross-linked polymers (Povidone), kaolin, calcium phosphate. These adsorbents do not appear to directly affect the control of the release of the active. Thus, such known compositions require the simultaneous presence of known gelling polymers, such as water-soluble or swellable cellulose derivatives. In addition thereto, such known solid compositions in tablet form are not gastro-resistant.

In other compositions known in solid form, the gastroresistance and/or the controlled/delayed release effect are instead only obtainable by suitable filming or external coating.

It is therefore felt the need to obtain solid compositions, so that the choice of components, both from a point of view of the relative *qualitative* combination, and from a *quantitative* point of view, are able to:

- effectively solubilize active lipophilic ingredients that are: poorly soluble in water and that, despite having good intestinal permeability, undergo rapid metabolism in the enterocyte and/or at the hepatic level (BCS II actives); or poorly soluble in water and that also show low intestinal permeability (BCS IV actives); and

- overcome the gastric lumen without any dissolution, or with negligible superficial erosion, in any case such as not to disintegrate the solid system, even in the presence of pepsin; and

- disintegrate progressively and slowly in the enteric environment, promoting a gradual and delayed release of the at least one active lipophilic ingredient; and

- lead to the formation of mixed micelles in the enteric environment, once in contact with enteric liquids, for example with bile salts (extemporaneous emulsion formation); and

- obtain a physically *stable* solid composition, which can, i.e., be further processed, for example for the preparation of tablets, despite the presence of components of an *oily* nature, for example without witnessing *leakage* phenomena.

SUMMARY OF THE INVENTION

A first object of the invention is a gradual enteric release oral solid composition comprising

i) at least one active lipophilic ingredient belonging to BCS class II and/or IV according to the biopharmaceutical classification system,

ii) a self-emulsifying vehicle comprising or consisting of

ii-a) a solubilizing oily matrix comprising or consisting of

- an ester of polyoxyethylene sorbitan with a fatty acid, the fatty acid being saturated or unsaturated and having a number of carbon atoms comprised between 16 and 22,

- a mono and diglyceride of a fatty acid, the fatty acid being saturated or unsaturated and having a number of carbon atoms comprised between 16 and 22,

- at least one unsaturated fatty acid having a carbon number comprised between 16 and 20,

ii-b) a caseinate derivative,

iii) suitable excipients and/or diluents,

wherein the ratio, expressed in formula (I), between the amount of caseinate derivative and the ratio of the amount of the least one polyoxyethylenated sorbitan ester and the amount of the least one active lipophilic ingredient

$$(I) \frac{quantity\ of\ the\ caseinate\ derivative}{\left(\frac{quantity\ of\ the\ at\ least\ one\ polyoxyethylene\ sorbitan\ ester}{quantity\ of\ the\ at\ least\ one\ lipophilic\ active\ ingredient}\right)}$$

is comprised between 0.03 and 0.6.

A second object of the invention is a process for preparing the oral solid composition, comprising the following steps:

a) preparing: the at least one active lipophilic ingredient; the polyoxyethylene sorbitan ester with a fatty acid; the mono and diglyceride of a fatty acid; the at least one unsaturated fatty acid; the caseinate derivative; suitable excipients and/or diluents;

b) dissolving or adsorbing the at least one active lipophilic ingredient in the oily matrix comprising the polyoxyethylene sorbitan ester, the mono and diglyceride of a fatty acid, the at least one unsaturated fatty acid, at a temperature comprised between 40°C and 100°C, to obtain a melted oily mixture;

c) adsorbing the melted oily mixture of step (b) above on the caseinate derivative, to obtain the vehicle in the form of a wet powder;

d) adding the vehicle and the suitable excipients and/or diluents.

The solid composition is for use in the prevention and/or treatment of a disease selected from the group consisting of: osteopenia; osteoporosis; cardiovascular disease; hypertension; venous insufficiency; oncological disease; and combinations of the foregoing.

A further object of the invention is the use of the oral solid composition for disorders occurring concomitantly with or caused by menopause, and/or for chronological and photo-ageing of the skin.

***Advantages of the invention***

In the following, a delivery system of lipophilic lipid-based self-emulsifying actives in the form of a solid gastro-resistant composition is described, capable of increasing the bioavailability and permeability of such active lipophilic ingredients through the intestine, and, if necessary, of reducing their pre-systemic metabolism.

The solid composition of the invention meets the needs of the prior art, as it is *overall* capable of:

- *effectively solubilizing active lipophilic ingredients.*

The solid composition solubilizes active lipophilic ingredients belonging to BCS class II and/or IV of the biopharmaceutical classification system, i.e. actives that are poorly soluble in water. The Applicant has selected a precise combination of components of the oily matrix such as to promote the complete solubilization of the at least one active lipophilic ingredient. It should be noted that some of the components of the oily matrix contribute to the *effective* solubilization of the at least one active lipophilic ingredient, while others can only be considered as modest solubilizers of the at least one active lipophilic ingredient. Despite this, the presence of components of the oily matrix, which represent modest solubilizers of the at least one active lipophilic ingredient, is essential to lead to the formation of eutectic mixtures within the oily matrix, thus lowering the melting temperatures; they are also essential for the extemporaneous generation of mixed micelles in the enteric environment, as will be explained below.

Again, it is noted that an effective solubilization of the at least one active lipophilic ingredient affects the bioaccessibility of the same active in the intestinal context, as will be confirmed below. The Applicant therefore considers that the presence of all components of the solubilising oily matrix is essential for this purpose. For the same purpose of increasing bioaccessibility, of particular importance is the *quantitative* aspect, linked to the weight ratio between the polyoxyethylene sorbitan ester and the at least one active lipophilic ingredient that appears in the ratio of formula (I) indicated in claim 1.

- *overcome the gastric lumen without any dissolution, or with negligible superficial erosion, such as not to disintegrate the solid system.*

It should be noted that, as effectively demonstrated in the Examples section (subsection 5.2.3), the solid composition does not disintegrate in the gastric environment; in fact, pepsin, while interacting with the caseinate derivative at the surface level of the solid composition, is *not* able to hydrolyze the caseinate derivative at the innermost part of the solid composition, thus preserving its overall integrity in the gastric fluid. To achieve this effect, it is also important to use a certain amount of caseinate derivative. From this, the Applicant considers that the ratio of formula (I) indicated in claim 1 is essential, which, in fact, includes the amount of the caseinate derivative;

- *disintegrating progressively and slowly in the enteric environment, promoting a gradual and delayed release of the at least one active lipophilic ingredient.*

The Applicant has demonstrated in the Examples section (subsections 5.2.2 and 5.2.4) that it is the combination of the oily matrix and the caseinate derivative, therefore the presence of the *vehicle as a whole,* that modulates the disaggregation time in the enteric context.

The Applicant has found that the disaggregation time in the enteric context is a function of the ratio of formula (I) set out in claim 1. In particular, there is a linear correlation ($R^2$=0.99) between the ratio of formula (I) and the disaggregation time. This correlation suggests that the higher the ratio (amount of the polyoxyethylene sorbitan ester/amount of the at least one active lipophilic ingredient) (referred to as "formula (II) ratio" in the following), the lower the amount of the caseinate derivative, the faster the breakdown of the solid composition.

Interestingly, although there is the presence of some components, such as the polyoxyethylene sorbitan ester, which could have led to an *early* disaggregation of the solid composition in an enteric environment, due to its water attraction properties (typical of surfactants), the solid composition has been shown overall to resist, in the sense that the presence of the caseinate derivative competes with some components of the oily matrix, acting as an agent promoting the gradual/delayed release of the active lipophilic ingredient in the intestine.

Still, the solid composition promotes the permeability of BCS II and/or IV active lipophilic ingredients.

Specifically for the BCS II active lipophilic ingredients, therefore with poor solubility in water, but high intestinal permeability, the experiments conducted, referred to in subsections 5.2.5 and 5.2.6, demonstrate that the claimed technology retains the good bioaccessibility (solubility in the aqueous fraction of the digest) of the BCS II active lipophilic ingredient, but breaks down its biotransformation into the enterocyte. Thus, the solid composition reduces extrusion of the at least one active lipophilic ingredient from the intestinal cell.

What affects the improvement or preservation of bioaccessibility is the effective solubilization of the at least one active lipophilic ingredient in the oily matrix, as already indicated above.

**In** the enteric context, the caseinate derivative is gradually hydrolysed by the proteolytic enzymes of pancreatin, resulting in a regular release of the at least one active lipophilic ingredient, which release is, among other things, *independent* of the concentration of the active itself.

- *leading to the formation of mixed micelles in the enteric environment.*

The solid composition has overall a *self-emulsifying* nature, i.e. it leads to the spontaneous formation of an oil-in-water emulsion (mixed micelles or micellar dispersion) only once in contact with enteric liquids, e.g. with bile salts (extemporaneous emulsion formation).

**In** this sense, the presence of the components of the solubilising oily matrix is essential. **In** particular, the polyoxyethylene sorbitan ester and the unsaturated fatty acid, which are surfactants, "draw" or attract water in the enteric context; in parallel, the mono- and diglycerides of a fatty acid are instead essential for the interaction with bile salts, leading to the generation of mixed micelles. The mono- and diglycerides also induce amorphization of the at least one active lipophilic ingredient, indicating that the at least one active lipophilic ingredient has actually reacted with the oily matrix.

There is no precipitation of the at least one active lipophilic ingredient once the enteric aqueous medium is reached, since the active ingredient interacts with the components of the oily matrix.

Micellar disaggregation and dispersion in the intestine is "activated" *in vivo* by enteric proteases (e.g., pancreatin).

Another technological difficulty linked to the formation of the solid composition of the invention resides in the presence of an oily matrix which must come into intimate contact with the caseinate derivative and with the other suitable excipients and/or diluents. Such fatty or oily matrices usually impact the stability of the solid compositions (for example, specifically for tablets, they could impact the hardness, which tends to decrease, and/or the friability of the tablets, which tends to increase, thus compromising the overall compatibility with industrial production and blistering processes). However, this difficulty has been overcome for the present invention by virtue of the qualitative-quantitative balance of the solid composition and/or the related preparation process.

The Applicant emphasizes that, although lipid-based self-emulsifying technologies are known, and although all the components included in the oral solid composition are known per se, the peculiarity of the invention lies in having achieved a precise *qualitative-quantitative* combination within the self-emulsifying vehicle to obtain all the aforementioned properties *overall,* finely balancing and compensating the various effects brought by each component included in the vehicle.

**DESCRIPTION OF THE FIGURES**

*Figure 1*: Chemical structure of Resveratrol (RES).

*Figure 2:* Images of Caco2 Cells; in particular, image (A) is acquired by SEM and illustrates Caco-2 microvilli, while image (B) is acquired by confocal microscope, in which it is possible to visualize the tight junctions (ZO-1) and the core.

*Figure 3:* Standard calibration curve with reference to subsection 5.1.8 of the Examples, constructed by diluting resveratrol in MeOH, to obtain final concentrations of calibrated solutions in the range of 0.5 to 150 $\mu$g/mL.

*Figure* 4: with reference to subsections 5.2.1 and 5.2.3 of the Examples, the image shows the formation of breaks on the surface of tablets containing RES-LiBADDS (bottom right) and tablets containing NaC (Sodium Caseinate) (top right) in *FSSGF/pepsin* medium. The surface of the FSSGF-disaggregated tablets *in the absence of pepsin* showed no breakage (top and bottom left). This is probably due to the hydrolytic activity of pepsin on NaC on the surface of the tablet.

*Figure 5:* with reference to subsections 5.2.2. and 5.2.3 of the Examples, the graph of the breakdown time as a function of NaC content. It is noted that, in the range between 250 and 625 mg, the higher the NaC content, the shorter the disintegration time of the

tablets. This correlation is linear with $R^2=0.9998$, and the equation of the corresponding line was calculated. However, it should be noted that this disaggregation behavior is less between 125 and 225 mg of NaC and the tablets already disaggregate in FSSGF, therefore without gastric resistance.

*Figure* 6: with reference to subsection 5.2.2 of the Examples, the graph shows a relationship between NaC content and disaggregation time in both FaSSIF models in the presence or absence of pancreatin. In the presence of pancreatin, a clear inverse linear correlation was observed between the NaC content and the disaggregation time;

in the absence of pancreatin, on the other hand, the correlation between the NaC content and the disaggregation time appeared direct. Furthermore, in the absence of pancreatin, with the sole exception of 250 mg of NaC, the disaggregation time was greater than 260 minutes.

*Figure* 7: with reference to subsection 5.2.4 of the Examples, the graph shows the disaggregation time of the tablets containing NaC compared to those containing LiBADDS with the same NaC content, specifically RES LiBADDS 013 vs 0.28 g NaC.

*Figure 8:* with reference to subsection 5.2.4 of the Examples, the graph shows the disaggregation time of the tablets containing NaC compared to those containing LiBADDS with the same NaC content, specifically RES LiBADDS 017 vs 0.50 g NaC.

*Figure 9:* with reference to subsection 5.2.4 of the Examples, the graph shows the disaggregation time of the tablets containing NaC compared to those containing LiBADDS with the same NaC content, specifically RES LiBADDS 019 vs 0.39 g NaC.

*Figure 10:* with reference to subsection 5.2.4 of the Examples, the graph shows the disintegration time of tablets containing LiBADDS 017, in the presence or absence of pancreatin.

*Figure 11:* with reference to subsection 5.2.4 of the Examples, the graph shows the disintegration time of tablets containing LiBADDS 019, in the presence or absence of pancreatin.

*Figure* 12: with reference to subsection 5.2.4 of the Examples, the graph shows the disintegration time of tablets containing LiBADDS 013, in the presence or absence of pancreatin.

*Figure* 13: with reference to subsection 5.2.4 of the Examples, the graph shows the disaggregation time of tablets containing LiBADDS in *FaSSIF+pancreatin.*

*Figure 14:* with reference to subsection 5.2.4 of the Examples, the graph of the disintegration time of the LiBADDS-containing tablets versus the NaC/(PS80/RES) ratio. A pseudo-linear correlation appears with $R^2=0.992$, suggesting a *direct* relationship between PS80/RES ratio and disaggregation time, and an *inverse* relationship between NaC and disaggregation time.

*Figure 15:* with reference to subsection 5.2.6. of the Examples, the graph represents the dissolution rate of RES from RES LiBADDS 019 over time. The pseudolinear trend ($R^2=0.999$) suggests a kinetics of order 0.

*Figure 16:* with reference to subsection 5.2.6. of the Examples, the graph shows the simulation of the Korsmayer-Peppas kinetic model ($R^2=0.982$).

DETAILED DESCRIPTION OF THE INVENTION

In the following, the invention and preferred embodiments thereof are described in more detail. It should be noted that, although the structure has been organised into paragraphs and sub-paragraphs, the information contained in each paragraph or subparagraph is not isolated, and is possibly combinable with that contained in other paragraphs or, more generally, with other information contained in the text of the patent application.

The term "at least" indicates a number equal to 1 or > 1, for example equal to 2 or 3.

*An oral solid and gradual enteric release composition*

As anticipated, the oral solid and gradual enteric release composition comprises

i) at least one active lipophilic ingredient belonging to BCS class II and/or IV of the biopharmaceutical classification system,

ii) a self-emulsifying vehicle comprising or consisting of

ii-a) a solubilizing oily matrix comprising or consisting of

- an ester of polyoxyethylene sorbitan with a fatty acid, the fatty acid being saturated or unsaturated and having a number of carbon atoms comprised between 16 and 22,
- a mono and diglyceride of a fatty acid, the fatty acid being saturated or unsaturated and having a number of carbon atoms comprised between 16 and 22,
- at least one unsaturated fatty acid having a carbon number comprised between 16 and 20,

ii-b) a caseinate derivative,

iii) suitable excipients and/or diluents,

wherein the ratio, expressed in formula (I), between the amount of the caseinate derivative and the ratio of the amount of the at least one polyoxyethylenated sorbitan ester and the amount of the active lipophilic ingredient

$$(I) \quad \frac{quantity\ of\ the\ caseinate\ derivative}{\left(\frac{quantity\ of\ the\ at\ least\ one\ polyoxyethylene\ sorbitan\ ester}{quantity\ of\ the\ at\ least\ one\ lipophilic\ active\ ingredient}\right)}$$

is comprised between 0.03 and 0.6.

The oral solid composition of the invention is a delayed release modulated *matrix.* Preferably, with reference to the solid composition, the relative gradual enteric release is a delayed intestinal release, preferably the transfer (or release) is activated by pancreatic protease enzymes. In other words, the solid composition is disaggregated in the intestine, preferably in the duodenum, by the pancreatic protease enzymes.

Preferably, the oral solid composition is gastro-resistant. The oral solid composition is further preferably resistant to pepsin in a gastric environment.

Preferably, the oral solid composition does not contain water, preferably with the exception of water inherently contained in some ingredients.

### *At least one active lipophilic ingredient (i)*

As anticipated, the solid composition comprises at least one active lipophilic ingredient (i) belonging to BCS class II or IV of the biopharmaceutical classification system.

The *Biopharmaceutics Classification System* (BCS) is a well-known method provided and approved by the *Food and Drug Administration* for the classification of drugs intended for gastrointestinal absorption. The method was designed in support of bioavailability and bioequivalence studies *[The Biopharmaceutics Classification System (BCS) Guidance, fda.gov, FDA].*

According to the conventions of the BCS, a substance is defined as high solubility when the highest immediate-release single dose is soluble in 250 mL of water, in a pH range of 1 to 7.5, while those substances for which the amount of drug absorbed is greater than 90% of the total dose administered are high permeability.

The active lipophilic ingredient is a BCS class II and/or IV active ingredient, in the sense that it can be

- a BCS II active lipophilic ingredient, i.e. an active ingredien characterised by low solubility and high permeability; and/or

- an active lipophilic ingredient of BCS class IV, i.e. an active characterized by low solubility and low permeability.

By active lipophilic ingredient is meant an active substance or compound which has a biological activity, and which is soluble in a fat/lipid, and/or in an oily or lipid mixture, and/or in an organic solvent, at room temperature.

Preferably, the active lipophilic ingredient has a logP value > 1 and < 4, preferably > 2 and < 3.5.

Preferably, the active lipophilic ingredient has a low solubility in water or aqueous medium, preferably this is < 0.05 mg mL$^{-1}$, preferably $\leq$ 0.03 mg mL$^{-1}$.

Preferably, the active lipophilic ingredient is a *small molecule.*

Preferably, the active lipophilic ingredient has a molecular weight comprised between 100 and 1000 g/mol, preferably between 100 and 800 g/mol, preferably between 100 and 500 g/mol, preferably between 100 and 400 g/mol, preferably between 100 and 300 g/mol, preferably between 100 and 250 g/mol, preferably between 100 and 200 g/mol.

Preferably, the at least one active lipophilic ingredient is resveratrol and/or boswellia and/or bromellin and/or a fat-soluble vitamin and/or a fat-soluble active ingredient soluble in the oily mixture.

Although the Examples were conducted with the use of the active lipophilic ingredient resveratrol, belonging to the BCS II class, the Applicant considers that any active lipophilic ingredient of class II and/or class IV, can be effectively conveyed, as long as it falls into the aforementioned BCS classes and is soluble in the oily matrix.

Preferably, the at least one active lipophilic ingredient is selected from the group consisting of: resveratrol, boswellia, bromellin, and combinations of the foregoing.

An example of the at least one active lipophilic ingredient is boswellia in combination with bromellin, or is resveratrol, preferably trans-resveratrol.

Preferably, the at least one active lipophilic ingredient is in an amount comprised between 0.5% and 5.0% by weight, preferably between 0.5% and 3% by weight, preferably between 0.5% and 2.7% by weight, preferably between 0.7% and 2.7% by weight, preferably between 1% and 2.7% by weight, preferably between 1.2% and 2.7% by weight, preferably between 0.7% and 1.2% by weight, preferably equal to 0.7%, or 1.2%, or 2.7% by weight, or 5.0% by weight, of the total weight of the composition.

Preferably, when the active lipophilic ingredient is resveratrol, the relative amount is comprised between 0.2% and 5.0% by weight, preferably between 0.2% and 3.5% by weight, preferably between 0.5% and 3% by weight, preferably between 0.5% and 2.5% by weight, preferably between 0.5% and 1.5% by weight, preferably between 0.5% and 1% by weight, preferably equal to 0.5%, or 1%, or 2.5% by weight, or 5.0% of the total weight of the composition.

Preferably, the solid composition also comprises a fat-soluble vitamin.

Preferably, the fat-soluble vitamin is selected from the group consisting of: Vitamin A, Vitamin E, Vitamin D, Vitamin K, and combinations of the foregoing.

Preferably, the fat-soluble vitamin is Vitamin E, in the form of tocopherol and/or esters thereof (e.g., tocopheryl acetate). Vitamin E is preferably completely optional; preferably, it serves as an antioxidant active.

Preferably, the fat-soluble vitamin is in an amount comprised between 0.05% and 3% by weight, preferably between 0.05% and 2.7% by weight, preferably between 0.05% and 2.5% by weight, preferably between 0.05% and 1% by weight, preferably between 0.05% and 0.5% by weight, preferably between 0.1% and 0.3% by weight, preferably equal to 0.2% or 2.5% by weight, of the total weight of the composition.

*Self-emulsifying vehicle (ii)*

By vehicle is meant a combination of substances that promotes solubilization of the at least one active lipophilic ingredient and its release at a desired site.

By self-emulsifying vehicle (for brevity also defined as "emulsifying vehicle" in the following) it is meant that the vehicle is capable of leading to the formation of an oil-in-water emulsion (micelle or micellar dispersion) at the enteric level, *in situ,* i.e. once in contact with the enteric fluids containing bile salts.

Preferably, the self-emulsifying vehicle is gradual enteric release.

Preferably, the self-emulsifying vehicle (ii) is solid. The solubilizing oily matrix (ii-a) is preferably adsorbed onto the caseinate derivative (ii-b).

By gradual enteric release vehicle is meant that the vehicle is capable of progressively releasing the at least one active lipophilic ingredient once the enteric context is reached.

The self-emulsifying vehicle (ii) comprises or consists of

ii-a) a solubilizing oily matrix comprising or consisting of

- an ester of polyoxyethylene sorbitan with a fatty acid, the fatty acid being saturated or unsaturated and having a number of carbon atoms comprised between 16 and 22,

- a mono and diglyceride of a fatty acid, the fatty acid being saturated or unsaturated and having a number of carbon atoms comprised between 16 and 22,

- at least one unsaturated fatty acid having a carbon number comprised between 16 and 20,

ii-b) a casein derivative.

Preferably, the emulsifying vehicle (ii) does not contain water, preferably with the exception of water inherently contained in some ingredients.

Preferably, the *in situ* formed micelles have nanometer sizes.

*Solubilizing oily matrix (ii-a)*

By solubilizing oily matrix (hereinafter "oily matrix") is meant a combination of substances or excipients that are solubilized, as a *whole,* in a lipophilic substance or in a lipophilic solvent, and which is capable of solubilizing the at least one active lipophilic ingredient. That is to say, the at least one active lipophilic ingredient is solubilized (soluble) in the solubilizing oily matrix.

Preferably, the solubilizing oily matrix (ii-a) is lipid in nature.

Note that the oily matrix contributes to the acceleration of the disaggregation of the solid composition, compensating the retarding effect induced by the caseinate derivative.

The solubilizing oily matrix (ii-a) comprising or consisting of

- an ester of polyoxyethylene sorbitan with a fatty acid, the fatty acid being saturated or unsaturated and having a number of carbon atoms comprised between 16 and 22,

- a mono- and diglyceride of a fatty acid, the fatty acid being saturated or unsaturated and having a number of carbon atoms comprised between 16 and 22,

- a fatty acid being unsaturated and having a number of carbon atoms comprised between 16 and 20.

Preferably, the oily matrix is in an amount comprised between 2% and 15% by weight, preferably between 3% and 13% by weight, preferably between 3% and 12% by weight, preferably between 4.7% and 12% by weight, preferably between 4.7% and 12% by weight, preferably between 4.7% and 7.4% by weight, preferably equal to 4.7%, or 5.4%, or 7.4%, or 11.9% by weight, on the total weight of the *composition.*

Preferably, the oily matrix is in an amount comprised between 2% and 10% by weight, preferably between 3% and 10% by weight, preferably between 3% and 8% by weight, preferably between 3% and 7.5% by weight, preferably between 3.7% and 7.5% by weight, on the total weight of the *vehicle.*

Note that this amount is sufficient to promote the formation of the oil-in-water emulsion in the intestinal context.

Note that the solubilizing oily matrix (ii-a) is adsorbed on the caseinate derivative (ii-b).

As mentioned above, only some of the components of the oily matrix contribute to the *effective* solubilization of the at least one active lipophilic ingredient.

Others are only modest solubilizers of the at least one active lipophilic ingredient. Despite this, their presence is essential for the formation of the oily matrix, i.e. leading to eutectic mixtures, consequently lowering the melting temperatures of other components of the oily matrix; at the same time, they are fundamental for the extemporaneous formation of mixed micelles in the enteric environment.

For example, the active lipophilic ingredient resveratrol shows a low solubility in most vegetable oils and very modest in glycerides, but has an excellent solubility in polyoxyethylene sorbitan ester derivatives. The choice of these components for the oily matrix is functional to obtain eutectic mixtures, with the consequent lowering of the melting point of some components, and for the formation of mixed micelles *in situ,* in the enteric environment, at the duodenal level, in combination with bile salts.

Preferably, the melting temperature of the oily matrix is comprised between 40°C and 100°C, preferably between 50°C and 100°C, preferably between 50°C and 90°C, preferably between 60°C and 80°C, preferably equal to 80°C or 70°C.

It should be noted that the combination of the components of the oily matrix is essential to impart all the effects described in the subsection *Advantages* of the invention; for example, in the absence of the at least one unsaturated fatty acid and/or of the mono- and diglycerides of a fatty acid, it is not possible to achieve the formation of the mixed micelles; furthermore, in their absence, an oily matrix would not be obtained, inside which the at least one active lipophilic ingredient interacts in a molecular way with the same matrix.

Preferably, the oily matrix (ii-a) contains no water, preferably with the exception of water inherently contained in some ingredients.

*Ester of polyoxyethylene sorbitan with a fatty acid*

The solubilizing oily matrix contains the ester of polyoxyethylene sorbitan with a fatty acid (also referred to as "polyoxyethylene sorbitan ester"), the fatty acid

- being saturated or unsaturated, preferably unsaturated, and

- having a number of carbon atoms comprised between 16 and 22, preferably the number of carbon atoms is comprised between 16 and 20, preferably between 16 and 18, preferably equal to 18.

Preferably, the ester of the polyoxyethylene sorbitan with a fatty acid serves as a non-ionic surfactant. Preferably, the ester of the polyoxyethylene sorbitan with a fatty acid acts as a solubilizer for the at least one active lipophilic ingredient.

Preferably, the solid composition contains no other non-ionic surfactants, in addition to the polyoxyethylene sorbitan ester.

It should be noted that the polyoxyethylene sorbitan ester, in addition to constituting a solubilizing agent for the at least one active lipophilic ingredient, is also able to accelerate the disaggregation, thus reducing the disaggregation times and competing, in this sense, with the caseinate derivative.

Preferably, the fatty acid of the polyoxyethylene sorbitan ester is an oleic acid.

Preferably, the ester of the polyoxyethylene sorbitan with a fatty acid is selected from the group consisting of: polysorbate 20, polysorbate 40, polysorbate 60, polysorbate 80, and combinations of the foregoing.

Preferably, the polyoxyethylene sorbitan ester with a fatty acid is polysorbate 80.

Preferably, the polyoxyethylene sorbitan ester with a fatty acid is in an amount comprised between 2% and 8% by weight, preferably between 2% and 7% by weight, preferably between 2% and 6.5% by weight, preferably between 2% and 6% by weight, preferably between 3.4% and 6% by weight, preferably between 5% and 6% by weight, preferably between 3.4% and 5% by weight, preferably equal to 3.4%, or 5%, or 6% by weight, of the total weight of the *composition.*

Preferably, the amount of polyoxyethylene sorbitan ester is comprised between 1% and 6% by weight, preferably between 1% and 5% by weight, preferably between 1.2% **and 5% by** weight, preferably between 2% and 5% by weight, preferably between 2.5% and 4% by weight, on the total weight of the *vehicle.*

Preferably, the amount of polyoxyethylene sorbitan ester is comprised between 30% and 75% by weight, preferably between 35% and 75% by weight, preferably between 40% and 73% by weight, on the total weight of the *oily matrix.*

Note that an amount higher than the maximum indicated for the polyoxyethylene sorbitan ester could induce an early disaggregation of the solid composition in the enteric context, not adequately balancing the retarding effect of the caseinate derivative.

It should be noted that an amount lower than the minimum indicated for the ester of the polyoxyethylene sorbitan would not be effective for the solubilization of the at least one active lipophilic ingredient, nor for the effect of compensation of the disaggregation delaying activity carried out by the caseinate derivative.

Preferably, the polyoxyethylene sorbitan ester is in the form of a liquid, preferably a viscous liquid, at room temperature.

Preferably, the polyoxyethylene sorbitan ester is hydrophilic, thus dispersible in water.

Preferably, the polyoxyethylene sorbitan ester has an HLB > 10, preferably > 12, preferably > 12 and $\leq$ 17, preferably > 14 and $\leq$ 17, preferably > 14 and $\leq$ 15.

Preferably, the quantitative ratio of formula (II) between the amount of the polyoxyethylene sorbitan ester and the following at least one active lipophilic ingredient

$$(II) \quad \frac{\textit{quantity of the polyoxyethylene sorbitan ester}}{\textit{quantity of the at least one lipophilic active ingredient}}$$

is comprised between 0.3 and 9, preferably between 0.5 and 9, preferably between 1.5 and 9, preferably between 2 and 9, preferably between 3.5 and 9, preferably between 3.5 and 7, preferably between 5 and 9, preferably between 2 and 9, preferably between 2 and 7, preferably between 3.5 and 5, preferably equal to 2, or to 3.5, or to 5, or to 6.8, or to 7, or to 9.

This ratio of formula (II), which is included in the ratio of formula (I), is functional to an optimal solubilization of the at least one active lipophilic ingredient, poorly soluble in water/aqueous medium, an aspect that affects the bioaccessibility of the same active in the enteric context, as well as the disaggregation time.

*Mono and diglyceride of a fatty acid*

The solubilizing oily matrix contains a mono- and diglyceride of a fatty acid (hereinafter "mono- and diglyceride of a fatty acid"), the fatty acid being

- saturated or unsaturated, preferably saturated, and

- having a number of carbon atoms comprised between 16 and 22, preferably the number of carbon atoms is comprised between 18 and 22, preferably between 20 and 22, preferably equal to 22.

The mono- and diglyceride of a fatty acid contributes to the formation of the emulsion in the enteric context, through the generation of mixed micelles with bile salts. **In** addition thereto, the mono- and diglyceride of a fatty acid determines the amorphization of the at least one active lipophilic ingredient.

Preferably, the mono- and diglyceride of a fatty acid is glyceryl behenate (Glyceryl Behenate).

By mono- and diglycerides of a fatty acid is meant a mixture comprising both monoglycerides and diglycerides of a fatty acid.

Preferably, the composition of the invention contains substantially no triglycerides, in the sense that the triglycerides can be present in a negligible amount, for example comprised between 0% and 0.001% by weight, on the total weight of the mono- and diglyceride of a fatty acid.

Preferably, the mono and diglyceride of a fatty acid is in an amount comprised between 0.5% and 8% by weight, preferably between 0.5% and 7% by weight, preferably between 1% and 7% by weight, preferably between 2% and 7% by weight, preferably between 1% and 2% by weight, preferably equal to 1%, or 2%, or 6.7% by weight, on the total weight of the *composition.*

Preferably, the mono and diglyceride is comprised between 0.2% and 6% by weight, preferably between 0.23% and 5% by weight, preferably between 0.5% and 5% by weight, preferably between 0.7% and 4.5% by weight, on the total weight of the vehicle.

Preferably, the amount of mono and diglyceride of a fatty acid is comprised between 15% and 63% by weight, preferably between 15% and 60% by weight, preferably between 20% and 60% by weight, preferably between 20% and 58% by weight, on the total weight of the *oily matrix.*

The mono- and diglyceride of a fatty acid is an amphiphilic compound.

Preferably, the mono- and diglyceride of a fatty acid is in the form of a powder or a waxy solid. **In** particular, it is in the form of a powder at room temperature, but melts at a temperature of about 80°C; therefore, it solidifies again at room temperature in the form of a waxy solid. Note that the presence of the ester of polyoxyethylene sorbitan reduces its melting temperature (eutectic mixture).

*At least one unsaturated fatty acid*

The solubilizing oily matrix contains at least one unsaturated fatty acid, having a number of carbon atoms comprised between 16 and 20 (hereinafter "unsaturated fatty acid"), preferably the number of carbon atoms is comprised between 16 and 18, preferably the number of carbon atoms is equal to 18.

Preferably, the at least one unsaturated fatty acid has at least one unsaturation, preferably has two unsaturations, preferably has two conjugated double bonds, i.e. contiguous bonds not separated by methylene groups ($-CH_2-$).

Preferably, the at least one unsaturated fatty acid is a mixture of positional and geometric isomers of linoleic acid; preferably, the at least one unsaturated fatty acid is a conjugated linoleic acid.

Preferably, the at least one unsaturated fatty acid is in an amount comprised between 0.1% and 0.7% by weight, preferably between 0.2% and 0.6% by weight, preferably between 0.2% and 0.5% by weight, preferably between 0.2% and 0.4% by weight, preferably between 0.25% and 0.3% by weight, preferably between 0.3% and 0.4% by weight, preferably equal to 0.25%, or to 0.3%, or to 0.4% by weight, on the total weight of the *composition.*

Preferably, the at least one unsaturated fatty acid is comprised between 0.05% and 0.5% by weight, preferably between 0.1% and 0.5% by weight, preferably between 0.1% and 0.4% by weight, of the total weight of the *vehicle*.

Preferably, the amount of the at least one unsaturated fatty acid is comprised between 1% and 8% by weight, preferably between 1% and 7.5% by weight, preferably between 2% and 7.5% by weight, preferably between 2% and 6.5% by weight, on the total weight of the *oily matrix.*

Preferably, the at least one unsaturated fatty acid is in the form of an oil at room temperature.

The at least one unsaturated fatty acid is not soluble in water or aqueous medium.

The at least one unsaturated fatty acid acts as a co-surfactant. In particular, the at least one unsaturated fatty acid, at pseudo-neutral pH (compatible with the enteric context), is in the form of an ionic surfactant/surfactant.

Preferably, the ratio between the amount of the at least one unsaturated fatty acid (liquid oil) and the amount of the polyoxyethylene sorbitan ester (surfactant) is comprised between 0.02 and 0.10, preferably between 0.03 and 0.10, preferably between 0.04 and 0.09, preferably between 0.05 and 0.09, preferably between 0.05 and 0.088, preferably between 0.05 and 0.080.

This ratio influences the formation of the extemporaneous emulsion in the intestine.

*Caseinate derivative (ii-b)*

The self-emulsifying vehicle (ii) also comprises a caseinate derivative (ii-b).

Preferably, the caseinate derivative (ii-b) is solid.

Preferably, the caseinate derivative (ii-b) acts as an adsorbent for the solubilizing oily matrix.

The caseinate derivative contributes to the gastroresistance of the solid composition, in the sense that the solid composition does not dissolve in the gastric environment, both in the presence and in the absence of pepsin.

The caseinate derivative contributes to the progressive or gradual release of the at least one active lipophilic ingredient from the self-emulsifying vehicle itself in the enteric context. Gradual cleavage of the caseinate derivative occurs in the presence of the proteolytic enzymes. Solubilization of the caseinate derivative increases with hydrolysis.

In the enteric context, preferably in the duodenum, the caseinate derivative acts as

- adsorbing agent, and

- agent capable of imparting gastro-resistance to the solid composition, and

- controlled/delayed release agent, and

- surfactant; in this sense, the caseinate derivative is soluble in water or aqueous medium, preferably at pH values $\geq 6$ compatible with the enteric environment. In these conditions, the caseinate derivative recalls water.

Preferably, the caseinate derivative is selected from the group consisting of: sodium caseinate, potassium caseinate, calcium caseinate, and combinations of the foregoing.

Preferably, the caseinate derivative is sodium caseinate.

Sodium caseinate is a soluble milk protein that is widely used in food industry and pharmaceutical art to develop nano-emulsions, nano-micelles and other delivery systems due to its unique chemical-physical properties and versatility of use [7-9]. Sodium caseinate has amphiphilic characteristics thanks to the ionized carboxyl groups and the protein core that make it particularly suitable as an emulsifying agent. This technological hypothesis is based on the experiments described by Van Skype and Wang, according to which sodium caseinate coagulates at a low pH and not by pepsin [18,19].

Preferably, the casein derivative is in an amount comprised between 20% and 60% by weight, preferably between 30% and 60% by weight, preferably between 40% and 60% by weight, preferably between 40% and 55% by weight, preferably equal to 40% or 50% or 55% by weight, on the total weight of the *composition.*

Preferably, the caseinate derivative is comprised between 10% and 40% by weight, preferably between 20% and 40% by weight, preferably between 25% and 35% by weight, preferably between 30% and 35% by weight, preferably between 31% and 33% by weight, on the total weight of the *vehicle.*

Note that, below the minimum quantitative value, the caseinate derivative does not promote the gastroresistance effect; while, above the maximum value, the amount of the caseinate derivative is too high, so it would be unbalanced with respect to the amount of the polyoxyethylene sorbitan ester, causing an excessive prolongation of the disaggregation times in the enteric context.

Note that such concentrations contribute to the adsorbent effect of the caseinate derivative. Values outside these ranges do not allow the adsorbent effect to be achieved.

Preferably, the caseinate derivative is in a solid form, preferably it is in a powder or granule form.

Preferably, the solid composition does not comprise an outer coating or gastro-resistant film or filming.

By gastro-resistant outer coating or gastro-resistant film or filming is meant an outer layer, in the sense that the latter surrounds an innermost layer or core, the latter usually containing one or more active ingredients, consisting of one or more excipients capable of imparting gastro-resistance to the pharmaceutical preparation. By way of example, known excipients, capable of imparting gastro-resistance, may be: shellac, polymers derived from methacrylic acid (e.g., Eudragard).

The oral solid composition preferably consists of a *single* gastro-resistant matrix.

Preferably, the weight ratio between the solubilizing oily matrix and the caseinate derivative is comprised between 1:3 and 1:10.5, preferably between 1:3 and 10.2, preferably between 1:3 and 1:9, preferably between 1:3 and 1:8.5, preferably between 1:5 and 1:8.5.

*Suitable excipients and/or diluents (iii)*

The solid composition also contains suitable excipients and/or diluents (iii); these contribute to the formation of the solid preparation, but do not directly impact on the delayed control of the at least one active lipophilic ingredient.

Preferably, suitable excipients and/or diluents for the preparation of a solid composition are selected from the group consisting of: glidants; lubricating agents; hardness-promoting agents; disaggregation-promoting agents; bulking agents; flavouring agents; sweetening agents; and combinations of the foregoing.

Preferably, suitable excipients and/or diluents are selected from the group consisting of: silica, magnesium stearate, calcium phosphate, hydroxypropyl cellulose, microcrystalline cellulose; and combinations of the foregoing.

Preferably, the suitable excipients and/or diluents are in a *total* amount comprised between 30% and 70% by weight, preferably between 30% and 60% by weight, preferably between 30% and 55% by weight, preferably between 33% and 55% by weight, preferably equal to 35.4%, or 51.4%, or still 54.6% by weight, on the total weight of the composition.

Preferably, the amount of silica, if present, is comprised between 1.5% and 3%, preferably between 2% and 2.5% by weight, on the total weight of the composition.

Preferably, the amount of calcium phosphate, if present, is comprised between 17% and 28% by weight, preferably between 18% and 28% by weight, on the total weight of the composition.

Preferably, the amount of hydroxypropyl cellulose, if present, is comprised between 1.5% and 3%, preferably between 2% and 2.5% by weight, on the total weight of the composition.

Preferably, the amount of microcrystalline cellulose, if present, is comprised between 2% and 25% by weight, preferably between 5% and 25% by weight, preferably between 9% and 25% by weight, preferably between 10% and 25% by weight, on the total weight of the composition.

*Formula (I) Ratio*

The ratio, expressed in formula (I) (defined as "ratio of formula (I)"), between the amount of the casein derivative and the ratio between the amount of the at least one polyoxyethylene sorbitan ester and the amount of the at least one active lipophilic ingredient

$$(I) \quad \left( \cfrac{quantity\ of\ the\ caseinate\ derivative}{\cfrac{quantity\ of\ the\ at\ least\ one\ polyoxyethylene\ sorbitan\ ester}{qquantity\ of\ the\ at\ least\ one\ lipophilic\ active\ ingredient}} \right)$$

is comprised between 0.03 and 0.6.

Preferably, the formula (I) ratio is comprised between 0.03 and 0.5, preferably between 0.03 and 0.45, preferably between 0.03 and 0.30, preferably between 0.03 and 0.20, preferably between 0.03 and 0.10, preferably between 0.03 and 0.08, preferably between 0.04 and 0.08, preferably between 0.05 and 0.08.

Note that such a quantitative ratio is functional because

- it promotes the solubility of the at least one active lipophilic ingredient in the oily matrix;

- it prevents the dissolution of the solid composition in the gastric environment;

- it promotes the disintegration of the solid composition in the enteric environment, preferably in the presence of pancreatin;

- it promotes the disintegration of the solid composition in the enteric environment in a gradual and slow manner.

Note that the presence of the ester of the polyoxyethylene sorbitan may result in an early disaggregation of the solid composition due to its water attracting properties. In this sense, it should be noted that the weight ratio indicated above and the presence of the caseinate derivative are essential to avoid such early disaggregation, capable of promoting a gradual release on contact with enteric fluids.

Such a formula (I) ratio is essential to provide the at least one active lipophilic ingredient in a non-metabolized form in the intestine.

Preferably, the solid composition has a dissolution time comprised between 60 and 300 minutes (5 hours), preferably comprised between 60 and 240 (4 hours) minutes, preferably equal to 120 minutes (about 2 hours).

The solid composition preferably has an overall disintegration time in the enteric environment comprised between 80 and 120 minutes, preferably between 90 and 120 minutes, preferably of 90 minutes.

Preferably, the gradual enteric release of the at least one active lipophilic ingredient follows an order 0 dissolution kinetic profile.

Preferably, the dissolution of the solid composition in the simulated intestinal fluid, in the presence of pancreatin, follows an order 0 kinetic pattern.

Preferably, the disintegration in the enteric environment takes place ("activated") in the presence of the proteolytic enzymes, for example in the presence of pancreatin.

Preferably, the solid composition increases the intestinal permeability of the at least one active lipophilic ingredient by a factor of 2.

**Solid composition form**

Preferably, the solid composition is in a technological form selected from: powder, granulate, tablet, capsule, preferably it is in a tablet form.

The solid composition is preferably a gastro-resistant tablet. In this sense, the gastro-resistant tablet is able to pass the gastric context in a stable and unscathed manner, that is, without undergoing *substantial* structural alterations and/or disaggregations in contact with gastric fluids.

The oral solid composition is preferably a tablet consisting of a *single* gastro-resistant matrix, by virtue of the presence of the caseinate derivative; preferably, the oral solid composition is not a tablet with a double-layer or multilayer structure, or is not a structure in which a *core or inner core* and an outer coating, the latter surrounding the core, are distinguished.

Preferably the tablet hardness is comprised between 85 and 130 N, preferably between 90 and 120 N, preferably between 92 and 115 N, preferably between 92 and 113 N, preferably between 92 and 105 N.

It should be noted that *leakage* of the oily matrix is not observed during the preparation of the solid composition.

Preferably, the solid composition is in the form of a dietary supplement.

Food supplement means a formulation falling within the definition underlying the Directive 2002/46/EC as amended. In this regulation, food supplements are defined as: "foodstuffs the purpose of which is to supplement the normal diet and which are concentrated sources of nutrients, such as vitamins and minerals, or other substances with a nutritional or physiological effect, including, but not limited to, amino acids, essential fatty acids, fibre and various plants and herbal extracts, both single- and multi-compound, in pre-dosed forms".

### *Use of the solid oral composition in the form of a gastro-resistant tablet*

Preferably, the solid oral composition is for use in the prevention and/or treatment of a pathology selected from the group consisting of: osteopenia; osteoporosis, cardiovascular diseases; hypertension; venous insufficiency; oncological diseases; and combinations of the foregoing.

Preferably, the solid composition is for use in the prevention and/or treatment of a disorder or pathology selected from the group consisting of: osteopenia, osteoporosis, cardiovascular diseases, hypertension, venous insufficiency, oncological diseases; disorders occurring concomitantly with or caused by menopause, preferably disorders such as hot flashes and peripheral vascular disorders, and/or for cutaneous chrono- and photo-ageing; and combinations of the foregoing.

Preferably, cardiovascular diseases are understood to mean diseases selected from the group consisting of: coronary diseases; myocardial infarction; hypertension; and combinations of the foregoing.

Preferably, oncological diseases are understood to mean tumours.

A further object of the invention is the (non-medical) use of the solid composition for disorders occurring concomitantly with or caused by menopause and/or for chrono- and photo-ageing of the skin.

According to the Italian Ministry of Health, menopause is not a disease, but a physiological moment in a woman's life, which coincides with the end of her fertility.

Preferably, the disorders occurring concomitantly with or caused by menopause are selected from the group consisting of: hot flashes; peripheral vascular disorders; profuse sweating; palpitations; tachycardia; blood pressure fluctuations; sleep disorders; dizziness; vaginal dryness; genital itching; irritability; unstable mood; fatigue; anxiety; demotivation; concentration disorders; memory disorders; decreased sexual desire; and combinations of the foregoing.

Preferably, *hot flashes* are sudden sensations of increased body temperature.

Preferably, the peripheral vascular disorders include, for example, tingling and/or numbness.

Preferably, chronoageing is due to genetic factors and metabolic, hormonal processes, and leads to the production of free radicals responsible for skin cell ageing.

Preferably, photo-ageing is due to exposure to light and sun, since UV rays facilitate the formation of free radicals, skin spots and wrinkles, damaging cellular DNA and membranes, the complex of elastic fibers and surface microcirculation.

### *Process for preparing the solid composition*

Preferably, the process for preparing the solid composition comprises the following stages:

a) preparing: the at least one active lipophilic ingredient; the polyoxyethylene sorbitan ester with a fatty acid; the mono and diglyceride of a fatty acid; the at least one unsaturated fatty acid; the caseinate derivative; suitable excipients and/or diluents;

b) dissolving or adsorbing the at least one active lipophilic ingredient in the oily matrix comprising the ester of the polyoxyethylene sorbitan with a fatty acid, the mono and diglyceride of a fatty acid, the at least one unsaturated fatty acid, at a temperature comprised between 40 °C and 100 °C, preferably at a temperature comprised between 50 °C and 90 °C, preferably between 60 °C and 80 °C, preferably equal to 70 °C, to obtain a molten oily mixture, preferably a clear molten oily mixture and/or without undissolved lumps;

c) adsorbing the melted oily mixture of step (b) above onto the casein derivative, to obtain the vehicle in the form of a wet powder, preferably stir for at least 30 minutes, preferably for 30 minutes;

d) combining the vehicle and the appropriate excipients and/or diluents, preferably proceeding with compression.

Still preferably, the process for preparing the solid composition comprises the following stages:

a') preparing: the at least one active lipophilic ingredient; the polyoxyethylene sorbitan ester with a fatty acid; the mono and diglyceride of a fatty acid; the at least one unsaturated fatty acid; the caseinate derivative; suitable excipients and/or diluents;

b') dissolving or adsorbing the at least one active lipophilic ingredient in the oily matrix comprising the ester of the polyoxyethylene sorbitan with a fatty acid, the mono and diglyceride of a fatty acid, the at least one unsaturated fatty acid, at a temperature comprised between 40 °C and 100 °C, preferably at a temperature comprised between 50 °C and 90 °C, preferably between 60 °C and 80 °C, preferably equal to 70 °C, to obtain a molten oily mixture, preferably a clear molten oily mixture and/or without undissolved lumps;

c') *nebulizing or spraying,* using a special instrument known to the one skilled in the art, the melted oily mixture of the previous step (b) onto the casein derivative, to obtain the vehicle in the form of a wet powder;

d') combining the vehicle and the appropriate excipients and/or diluents, preferably proceeding with compression.

It should be noted that, for step (b) or (b') (of dissolving or adsorbing the at least one active lipophilic ingredient in the oily matrix), the Applicant also speaks of *adsorption* since there may be actives that are not completely soluble in the oily matrix.
Between the stages (c) of adsorption and (d) of bonding (or between the stages (c') of nebulization and (d') of bonding) allow the vehicle in powder form obtained from the stage (c) of adsorption to cool, preferably until it reaches room temperature; at the same time, preferably, leave the vehicle in powder form to stand for 2 hours.

EXAMPLES

Illustrative and non-limiting examples of the invention are given below.

***Example 1: Oral solid composition in the form of a tablet with gradual enteric release.***

| Phase | Components | Grams per tablet (dilution produced) | Grams per 1000 tablet |
|---|---|---|---|
| A | t-Resveratrol | 0.0050 | 5.01 |

| | | | |
|---|---|---|---|
| A | Compritol 888 ATO (Mono and Diglycerides of Behenic Acid) | 0.0100 | 10.00 |
| A | Tonalin A 80 (CLA) | 0.0030 | 2.50 |
| A | Polysorbate 80 (PS80) | 0.0340 | 33.50 |
| A | Tocopherol | 0.0020 | 2.00 |
| B | EMUL AC (NaC) | 0.4000 | 400.00 |
| D | Silica | 0.0250 | 25.00 |
| C | Magnesium stearate | 0.0250 | 25.00 |
| C | A Comprez (Tribasic calcium phosphate) | 0.2250 | 225.00 |
| C | Celny (hydroxypropyl cellulose) | 0.0250 | 25.00 |
| C | Ceolus KG802 (microcrystalline cellulose) | as needed 1 g | as needed 1000 g |
| | **Total** | **1.0000** | **1000.00** |

*Method of preparation:*

(Step A) Dissolve the mixture of Compritol ATO E, Polysorbate 80, Tonalin A 80 and Tocopherol at 80°C until a clear straw-coloured mixture is obtained. Proceed to disperse the resveratrol until it completely dissolves.

(Step B) Spray the lipid solution onto the stirred sodium caseinate in a horizontal mixer with auger and allow to stir for 30 minutes. Allow the powder to cool to room temperature and allow the powder to stand for further 2 hours.

(Step C) Next, combine the premixed Phase C excipients and restart the mixing for 30 minutes.

(Step D) Finally, add the silica and allow to stir for a further 15 minutes by activating the vacuum hood. Then proceed to compression and hardness, friability and abrasion tests.

Hardness (average of 10 tablets): 102 N $\pm$ 9 N

Crumbliness: test passed

Abrasion: test passed

**Example 2: Oral solid composition in the form of a tablet with gradual enteric release.**

| Phase | Components | Grams per tablet (dilution produced) | Grams per 1000 tablet |
|---|---|---|---|
| A | t-Resveratrol | 0.0050 | 5.01 |
| A | Compritol 888 ATO (Mono and Diglycerides of Behenic Acid) | 0.0200 | 20.00 |
| A | Tonalin A 80 (CLA) | 0.0040 | 4.00 |
| A | Polysorbate 80 (PS80) | 0.0500 | 50.0 |
| A | Tocopherol | 0.0020 | 2.00 |
| B | EMUL AC (NaC) | 0.4000 | 400.00 |
| D | Silica | 0.0250 | 25.00 |
| C | Magnesium stearate | 0.0350 | 35.00 |
| C | A Comprez (Tribasic calcium phosphate) | 0.2750 | 275.00 |
| C | Celny (hydroxypropyl cellulose) | 0.0250 | 25.00 |

| | | | |
|---|---|---|---|
| C | Ceolus KG802 (microcrystalline cellulose) | as needed 1 g | as needed 1000 g |
| | **Total** | *1.0000* | *1000.00* |

*Method of preparation:*

(Step A) Dissolve the mixture of Compritol ATO E, Polysorbate 80, Tonalin A 80 and Tocopherol at 80°C until a clear straw-coloured mixture is obtained. Proceed to disperse the resveratrol until it completely dissolves.

(Step B) Spray the lipid solution onto the stirred sodium caseinate in a horizontal mixer with auger and allow to stir for 30 minutes. Allow the powder to cool to room temperature and allow the powder to stand for further 2 hours.

(Step C) Next, combine the premixed Phase C excipients and restart the mixing for 30 minutes.

(Step D) Finally, add the silica and allow to stir for a further 15 minutes by activating the vacuum hood. Then proceed to compression and hardness, friability and abrasion tests.

Hardness (average of 10 tablets): 98 N ± 6 N

Crumbliness: test passed

Abrasion: test passed

**Example 3: Oral solid composition in the form of a tablet with gradual enteric release.**

| Phase | Components | Grams per tablet (dilution produced) | Grams per 1200 tablet |
|---|---|---|---|
| A | t-Resveratrol | 0.0300 | 3.01 |
| A | Compritol 888 ATO (Mono and Diglycerides of Behenic Acid) | 0.0800 | 80.00 |

| Phase | Components | Grams per tablet (dilution produced) | Grams per 1200 tablet |
|---|---|---|---|
| A | Tonalin A 80 (CLA) | 0.0030 | 3.00 |
| A | Polysorbate 80 (PS80) | 0.0600 | 60.00 |
| A | Tocopherol | 0.0020 | 2.00 |
| B | EMUL AC (NaC) | 0.6000 | 600.00 |
| D | Silica | 0.0250 | 25.00 |
| C | Magnesium stearate | 0.0250 | 25.00 |
| C | A Comprez (Tribasic calcium phosphate) | 0.2250 | 225.00 |
| C | Celny (hydroxypropyl cellulose) | 0.0250 | 25.00 |
| C | Ceolus KG802 (microcrystalline cellulose) | as needed 1.2 g | as needed 1200 g |
| | **Total** | **1.2000** | **1200.00** |

*Method of preparation:*

(Step A) Dissolve the mixture of Compritol ATO E, Polysorbate 80, Tonalin A 80 and Tocopherol at 80°C until a clear straw-coloured mixture is obtained. Proceed to disperse the resveratrol until it completely dissolves.

(Step B) Spray the lipid solution onto the stirred sodium caseinate in a horizontal mixer with auger and allow to stir for 30 minutes. Allow the powder to cool to room temperature and allow the powder to stand for a further 2 hours.

(Step C) Next, combine the premixed Phase C excipients and restart the mixing for 30 minutes.

(Step D) Finally, add the silica and allow to stir for a further 15 minutes by activating the vacuum hood. Then proceed to compression and hardness, friability and abrasion tests.

Hardness (average of 10 tablets): 94 N ± 5 N
Crumbliness: test passed
Abrasion: test passed.

**Example 4: Oral solid composition in the form of a tablet with gradual enteric release.**

| Phase | Components | Grams per tablet (dilution produced) | Grams per 1000 tablet |
|---|---|---|---|
| A | Vitamin K2 (50,000 ppm titer in MCT) | 0.025 (equal to 120 µg) | 25 |
| A | Compritol 888 ATO (Mono and Diglycerides of Behenic Acid) | 0.03 | 30 |
| A | Tonalin A 80 (CLA) | 0.004 | 4 |
| A | Polysorbate 80 (PS80) | 0.02 | 20 |
| A | Tocopherol | 0.004 | 4 |
| B | EMUL AC (NaC) | 0.55 | 550 |
| D | Silica | 0.03 | 30 |
| C | Magnesium stearate | 0.04 | 40 |
| C | A Comprez (Tribasic calcium phosphate) | 0.25 | 250 |
| C | Celny (hydroxypropyl cellulose) | 0.025 | 25 |
| C | Ceolus KG802 (microcrystalline cellulose) | as needed 1 g | as needed 1000 g |

| | Total | 1.0000 | 1000.00 |
|---|---|---|---|

*Method of preparation:*

(Step A) Dissolve the mixture of Compritol ATO E, Polysorbate 80, Tonalin A 80 and Tocopherol at 40°C until a clear straw-coloured mixture is obtained. Proceed to disperse the Vitamin K2 until it is completely dissolved.

(Step B) Spray the lipid solution onto the stirred sodium caseinate in a horizontal mixer with auger and allow to stir for 30 minutes. Allow the powder to cool to room temperature and allow the powder to stand for a further 2 hours.

(Step C) Next, combine the premixed excipients of Step C and restart mixing for 30 minutes.

(Step D) Finally, add the silica and allow to stir for a further 15 minutes by activating the vacuum hood. Then proceed to compression and hardness, friability and abrasion tests.

Hardness (average of 10 tablets): 104 N ± 9 N.

Friability: test passed.

Abrasion: test passed.

*Example 5: In vitro test*

*5.1. Preparations and methods*

*5.1.1. Preparation of NaC-Based Tablets (Sodium Caseinate)*

In a stainless steel capsule, 125 g of EMUL AC (Sodium Caseinate), 20 g of RES (Resveratrol), 575 g of Ceolus KG802 (microcrystalline cellulose), 25 g of amorphous silica, 200 mg of A Comprez (anhydrous dibasic calcium phosphate) and 25 g of magnesium stearate were weighed, mixed with a mechanical stirrer at 300 rpm and then mixed with a balloon for another 10 minutes. The powder mixture was sieved with a stainless steel sieve (40 mesh) and further compressed to obtain 1.0 g tablets. The tablets were subjected to quality controls for single-dose dosage forms, such as hardness, flowability, friability and abrasion.

The same method was employed to produce tablets containing 150, 200, 225, 250, 375, 437.5, 500, and 625 mg of NaC (Table 1, Tablets C2 to C9, respectively). The tablets were loaded with the same amount of excipients and the difference in NaC content was restored with microcrystalline cellulose.

Table 1: Composition of NaC-based tablets.

|  | *C1* | *C2* | *C3* | *C4* | *C5* | *C6* | *C7* | *C8* | *C9* |
|---|---|---|---|---|---|---|---|---|---|
| *RES (g)* | 0.020 | 0.020 | 0.020 | 0.020 | 0.020 | 0.020 | 0.020 | 0.020 | 0.020 |
| *EMUL AC (NaC) (g)* | 0.125 | 0.150 | 0.200 | 0.225 | 0.250 | 0.375 | 0.437 | 0.500 | 0.625 |
| *Industrial feasibility* | Yes | Yes | Yes | Yes | Yes | Yes | Yes | Yes | Yes |

*5.1.2. Evaluation of gastric strength of NaC-based tablets in FSSGF*

Six tablets containing 125 mg of NaC were inserted into the disaggregation device, according to the European Pharmacopoeia (EP). The disaggregation vessel was filled with 800 mL of FSSGF *(Fasted State Simulated Gastric Fluid)* at pH=1.6 in the presence of pepsin from porcine stomach at a concentration of 2000 U/ml [24-26]. The test was conducted for 2 hours at 37°C.

Testing was repeated *in the absence* of pepsin.

The same test was performed to evaluate the gastric resistance of the tablets containing 150, 200, 225, 250, 375, 437.5, 500, and 625 mg of NaC referred to in Table 1. The tablets were further tested with a machine capable of mimicking the mechanics of gastro-enteric movements *in the presence* of FSSGF-pepsin.

*5.1.3. Evaluation of the disaggregation of NaC-based tablets in FaSSIF*

Half of the FSSGF dispersion volume was added to 400 mL of 2X concentrated FaSSIF *(Fasted State Simulated Intestinal Fluid)* according to the two-step dissolution experiment described by Geboers et al. (Geboers S, Stappaerts J, Tack J, Annaert P, Augustijns P. In vitro and in vivo investigation of the gastrointestinal behavior of simvastatin. Int J Pharm. 2016 Aug 20;510(1):296-303. doi: 10.1016/j.ijpharm.2016.06.048). The pH was adjusted to 6.5 with sodium bicarbonate and the final mixture was added with pancreatin X 4 USP to the final concentration of 10 mg/mL.

The test was conducted until complete tablet disaggregation and then repeated *in the absence* of pancreatin X 4 USP.

*5.1.4. Preparation of RES tablets containing LiBADDS*

Several tablets were prepared by varying the mutual ratio between NaC (Sodium Caseinate), Polysorbate 80 (PS80) and RES (Resveratrol), keeping Glyceryl dibehenate (GDB, COMPRITOL 888 ATO) and conjugated linoleic carboxylic acid (CLAC, TONALIN A 80) constant (Table 2, tablets "RES LiBADDS 013, 015, 017 and 019").

Veremul T80 (PS80), Compritol 888 ATO, Tonalin A 80 and tocopherol (optional) were mixed in a 500 ml glass beaker and heated to 85°C to achieve a clear oily phase. The RES was weighed and poured into the warm oily phase under slight mechanical agitation. The mixture was stirred at 85°C with a thermostatic probe until complete solubilization of RES. Subsequently, the hot oily phase was rapidly poured into the stainless steel vessel containing EMUL AC under rapid mixing to obtain a uniform wet powder. As soon as the coarse powder reached 25°C, the remaining excipients, including calcium phosphate, magnesium stearate, microcrystalline cellulose and amorphous silica, were added under mixing to obtain 1000 g of final LiBADDS powder containing RES. The powder was further sieved and compressed to obtain the final tablets. The tablets were subjected to quality controls for single-dose pharmaceutical forms, such as hardness, flowability, friability and abrasion.

Among these, only 3 fully passed the quality controls and the overall industrial feasibility assessment (Table 2).

| | RES LiBADDS 013 | RES LiBADDS 015 | RES LiBADDS 017 | RES LiBADDS 019 |
|---|---|---|---|---|
| RES (g) | 0.010 | 0.010 | 0.010 | 0.005 |
| EMUL AC (NaC) (g) | 0.280 | 0.300 | 0.500 | 0.390 |
| VEREMUL T 80 (PS 80) (g) | 0.035 | 0.025 | 0.050 | 0.045 |
| COMPRITOL 888 ATO (GDB) (g) | 0.010 | 0.010 | 0.010 | 0.005 |
| TONALIN A 80 (CLAC) (g) | 0.005 | 0.005 | 0.005 | 0.0025 |
| Ratio (PS80/RES) | 3.5 | 2.5 | 5 | 9 |
| NaC/(PS80/RES) ratio | 0.080 | 0.120 | 0.100 | 0.043 |
| Industrial feasibility | Yes | Yes, but with some technical limitations | Yes | Yes |

Table 2. Qualitative-quantitative composition of RES LiBADDS tablets (the excipients for compression are the same as in Table 1).

*5.1.5. Evaluation of gastric resistance of RES tablets containing LiBADDS*

Six tablets of RES LiBADDS 019 were placed in the disaggregation device, as per EP. The chamber was filled with 800 mL of FSSGF at pH=1.6.

Testing was conducted for 2 hours at 37°C *in the presence and absence* of pepsin at a concentration of 2000 U/mL.

The same test was performed to evaluate the gastric resistance of the tablets containing 125, 150, 200, 225, 250, 375, 437.5, 500, and 625 mg of NaC referred to in Table 1.

### 5.1.6. Evaluation of the disaggregation of LiBADDS tablets containing RES in FaSSIF

Half the volume of the FSSGF dispersion was added to 400 mL of double concentrated FaSSIF, according to the two-step dissolution experiment described by Geboers et al (Geboers S, Stappaerts J, Tack J, Annaert P, Augustijns P. In vitro and in vivo investigation of the gastrointestinal behavior of simvastatin. Int J Pharm. 2016 Aug 20;510(1):296-303. doi: 10.1016/j.ijpharm.2016.06.048). The pH was adjusted to 6.5 with sodium bicarbonate and the final mixture was added with pancreatin X 4 USP to the final concentration of 10 mg/mL. Testing was conducted until complete tablet disaggregation.

The test was performed *in the presence and absence* of pancreatin.

At the end of the session, an aliquot of 20 mL was taken, centrifuged at 6000 rpm for 15 minutes and analyzed with HPLC coupled with DAD detector to titrate the RES isomers, both in the supernatant and in the pellet.

### 5.1.7. Bioaccessibility of RES in FSSGF/FaSSIF mixed micelle

The supernatants obtained after centrifugation were mixed with an equal volume of ethyl acetate to break the micelle and extract the RES in both *cis* and *trans* isomers. The organic step was then dried by rotary evaporation and replaced with 10 ml of methanol. The methanol solution was then analyzed by HPLC-DAD. The pellets collected on the bottom of the vials as previously described were mixed with the minimum volume of methanol and further analyzed with HPLC-DAD.

The bioaccessible fraction (BF) of RES was calculated according to the following equation (1):

$$(1)\ BF = (RES_{mic}/\ RES_{tot})*100$$

wherein: BF is the bioaccessible fraction, $RES_{mic}$ is the RES fraction encapsulated in the supernatant mixed micelle, and $RES_{tot}$ is the total RES loaded into the tablets recovered from the HPLC-DAD analysis.

### 5.1.8. Dissolution test

In vitro release of RES from LiBADDS 019 RES tablets was performed in FaSSIF in the presence of pancreatin (10 mg/ml) (900ml, pH=6.5) at 37°C $\pm$ 0.50 °C and 100 rpm, using the USP II dissolution test apparatus *(P Dissolution test Apparatus, Model 1912, SunShine Scientific,* New Delhi, India). A 10 ml aliquot was taken from the dissolution medium at specified time intervals (15', 30', 45', 60', 90', 120' and complete dissolution) and immediately replenished with the same volume of FaSSIF+pancreatin maintained at 37°C. The liquid aliquots were then centrifuged at 6000 rpm for 15 minutes. Both the supernatants and the pellet were further analyzed spectrophotometrically (HPLC-DAD) after extraction with ethyl acetate.

An Agilent 1200 HPLC system (Waldbronn, Germany) was equipped with an *online* mobile phase degasser, a quaternary pump, an autosampler, a column heater and a diode detector (DAD) as described below.

| |
|---|
| ***Column:*** Gemini® C18 analytical column (150 x 2.0 mm i.d., 5 $\mu$m, Phenomenex, Torrance, CA, USA) |
| ***Temperature of the column:*** 30°C |
| ***Flow:*** 0.3 mL/min |
| ***Volume of injection:*** 2 $\mu$L |
| ***DAD Signal:*** 310 nm for *trans*-resveratrol and 280 nm for *cis*-resveratrol |
| ***Mobile Phase Method:*** |
| MeOH: acetic acid: H$_2$O (45:5:50 v/v%) |
| 1.2 v/v% acetic acid in MeOH |
| LOQ: 0,04 microg/ml |
| LOD: 0,14 microg/ml |

(continued)

| Time (min) | A (%) | B (%) |
|---|---|---|
| 0 | 100 | 0 |
| 6 | 90 | 10 |
| 7 | 0 | 100 |
| 7.50 | 0 | 100 |
| 8 | 100 | 0 |
| 10 | 100 | 0 |

Total run time = 14 minutes.

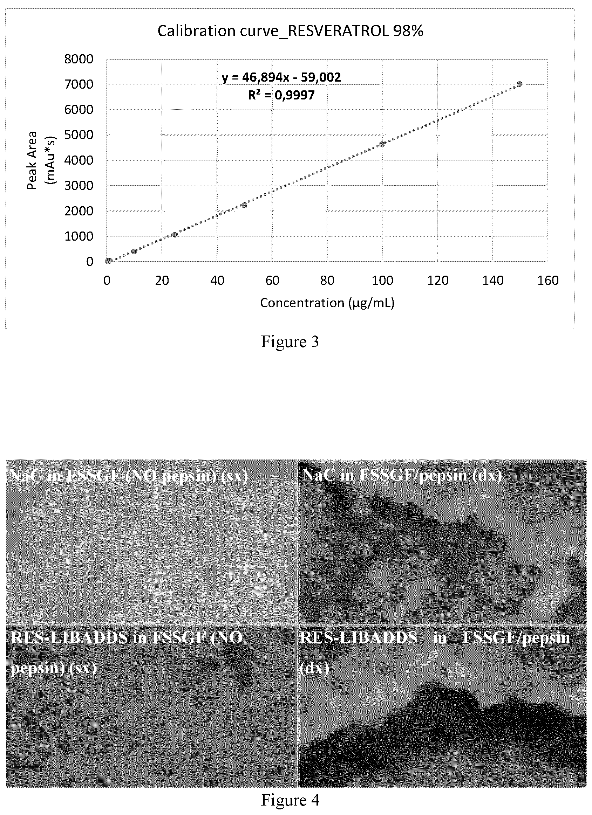

The standard curve was constructed by diluting resveratrol in MeOH, to obtain final concentrations of calibrated solutions in the range between 0.5 and 150 μg/mL (calibration curve in Figure 3).

### 5.2. Results

#### 5.2.1. Gastric resistance ofNaC based tablets in FSSGF

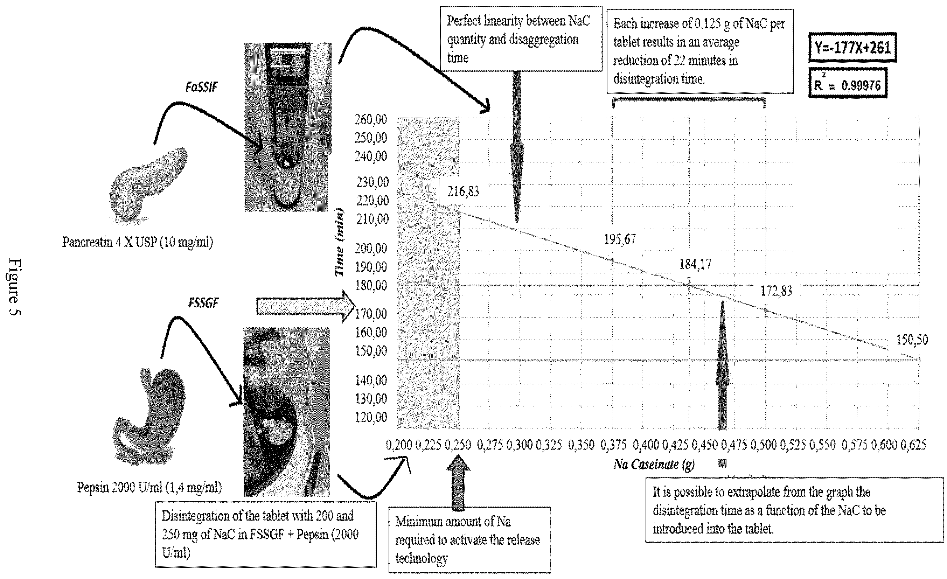

All tablets, except those containing 125, 150, 200 and 225 mg of NaC, as shown in the graph of Figure 5, passed both gastric resistance tests.

The tablet surface was analyzed under light microscope *in the presence and absence* of pepsin to assess whether the enzyme changed the morphology of the tablet surface.

As shown in Figure 4, the presence of fractures and an overall jagged appearance of the surface of the FSSGF-disaggregated tablets can be detected *in the presence* of pepsin. This characteristic was completely absent in the disaggregated tablets *in the absence* of pepsin. This evidence is the evidence that pepsin changed the surface morphology of the tablets, partially hydrolysing the NaC and creating breaks.

It is important to note, however, that overall, the FSSGF-disaggregated tablets, *both with and without pepsin,* passed the gastric endurance test according to EP.

#### 5.2.2. Disaggregation profile of NaC-based tablets in FaSSIF

Tablets containing 250, 375, 437.5, 500 and 625 mg of NaC were disaggregated in FaSSIF in the presence and absence of 4 X USP pancreatin. The average tablet disaggregation time was plotted as a function of NaC content in an attempt to discover a relationship.

Interestingly, *in the presence* of pancreatin, an inverse linear correlation was identified ($R^2$=0.9998), as illustrated in Figure 5, where the graph indicates that each 125 mg increase in NaC in the tablet led to a reduction in disaggregation time of 22 minutes. These data suggest a behavior similar to the "delayed release" mediated by NaC, activated *in vivo* by enteric proteases.

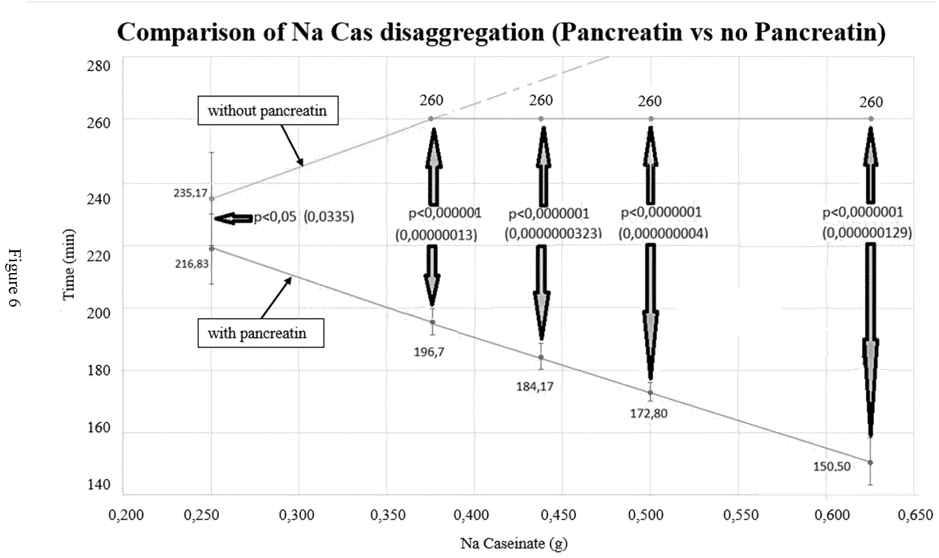

The graph in Figure 6 instead plots the disaggregation time of the tablets with respect to the NaC content. It is noted that, *in the absence* of pancreatin, there is a direct correlation between the NaC content and the disaggregation time, with a considerable increase of the latter. In fact, *in the absence* of pancreatin, with the sole exception of the tablet containing 250 mg, the disintegration time was greater than 260 minutes, which represents the threshold of session duration (Figure 6). This data confirms the role of pancreatin in the cleavage of NaC molecules, thereby accelerating tablet disaggregation.

In addition, it appears that the pseudo-neutral pH of the FaSSIF disaggregation medium, although capable of maintaining NaC in soluble ionized form (pH>pKa of NaC), is not sufficient to ensure faster disaggregation. A likely explanation could be the presence of NaC salification on the *surface* of the tablet, but *not throughout its inner core,* so the presence of pancreatin is to be considered fundamental to promote disaggregation.

#### 5.2.3. Gastric resistance of LiBADDS tablets containing RES

The four tablets that passed the quality controls and the industrial feasibility assessment (see subsection 5.1.4) passed the gastric resistance test.

The surface of the tablet was analysed under an optical microscope *in the presence and absence* of pepsin, to assess whether the enzyme has changed the morphology of the tablet surface.

As shown in Figure 4, the presence of fractures and an overall jagged appearance of the FSSGF/pepsin disaggregated tablets can be detected. This characteristic is completely absent in FSSGF-disaggregated tablets *in the absence of* pepsin. This suggests that pepsin changed the surface morphology of the tablets by partially hydrolyzing the NaC and creating breaks.

However, tablets disaggregated in FSSGF, *both with and without pepsin,* passed gastric resistance tests assessed based on EP and a machine that mimics gastro-enteric movement (Figure 5).

Overall, these data indicate that pepsin, while interacting with NaC on the tablet surface, is *not* able to hydrolyze NaC throughout the inner core, thus preserving its overall integrity in FSSGF.

Interestingly, although the PS80 could have led to an early break-up of the tablet, due to its water-attractive properties, the structure proved to be resilient overall.

### 5.2.4. Disaggregation of LiBADDS-Containing RES Tablets in FaSSIF

The adsorption of the LiBADDS phase on the solid excipients of the tablets entails several problems in terms of compressibility. In particular, the fat step reduces the hardness and increases the friability of the tablets, thus compromising the overall compatibility with the industrial production and blistering process.

The main objective to be achieved during the manufacturing process is the complete solubilization of the RES in the oil step. RES has a low solubility in most vegetable oils and very modest in glycerides, but shows an excellent solubility in PS80, especially at high temperature. Most likely, the best micellization in the intestine should occur if the RES is well solubilized in the lipid-based self-emulsifying step. This physical phenomenon, in fact, creates the conditions for the active interaction of RES resin with the components of the matrix.

The three tablets that completely passed the quality control and industrial manufacturing (RES LiBADDS 013, 017 and 019) and the one that passed them with some limits (RES LiBADDS 015) (see Table 2 of subsection 5.1.4) were tested both in the presence and in the absence of pancreatin 4 X USP, according to the method described in the previous paragraphs.

Unlike the results obtained with NaC-containing tablets, the association between LiBADDS phase and NaC changed the disaggregation behavior. In fact, increasing the NaC content in LiBADDS-based tablets appears to prolong the disintegration time. This is in contrast to the data seen for tablets containing NaC.

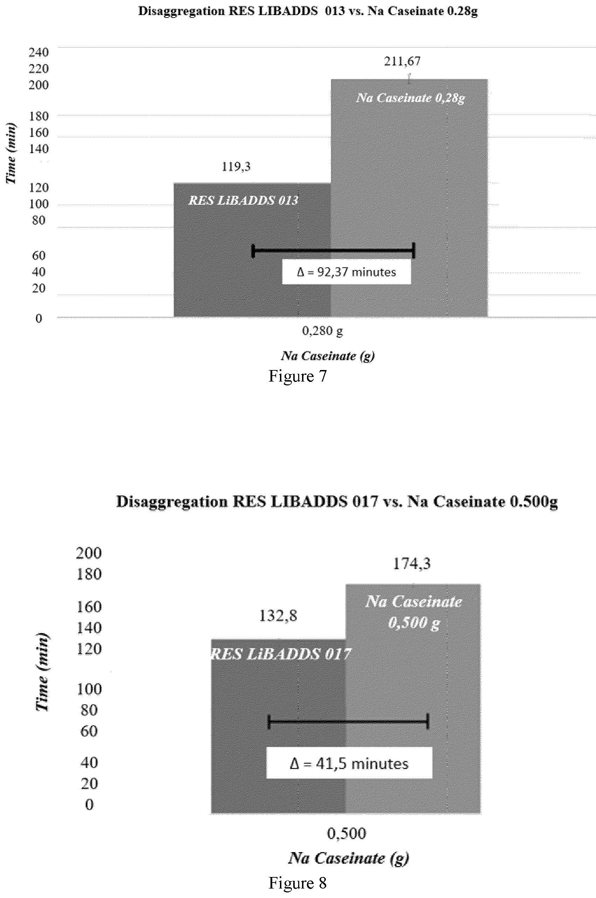

In all cases, the mean disaggregation time was lower for LiBADDS-containing tablets than those containing NaC, establishing the role of the LiBADDS step in accelerating disaggregation (Figures 7-9 and 13).

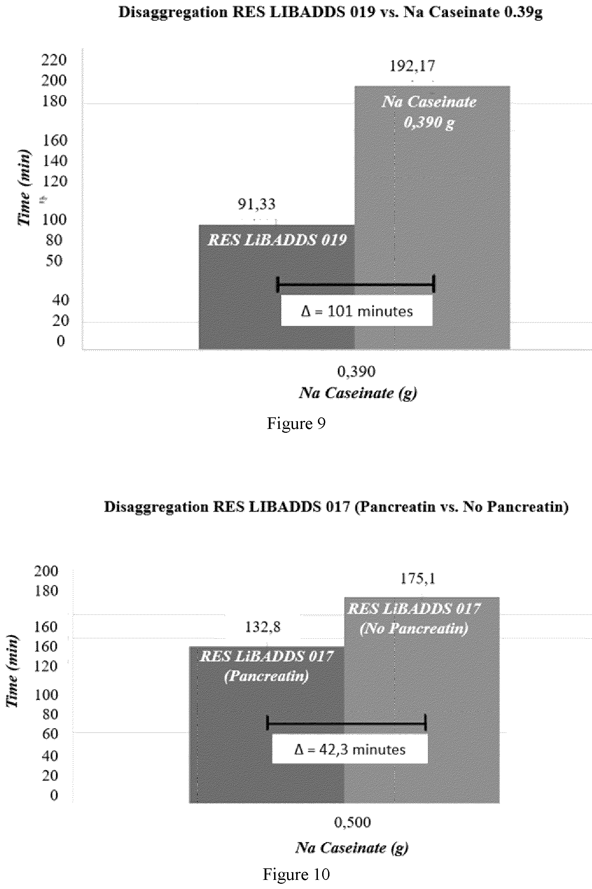

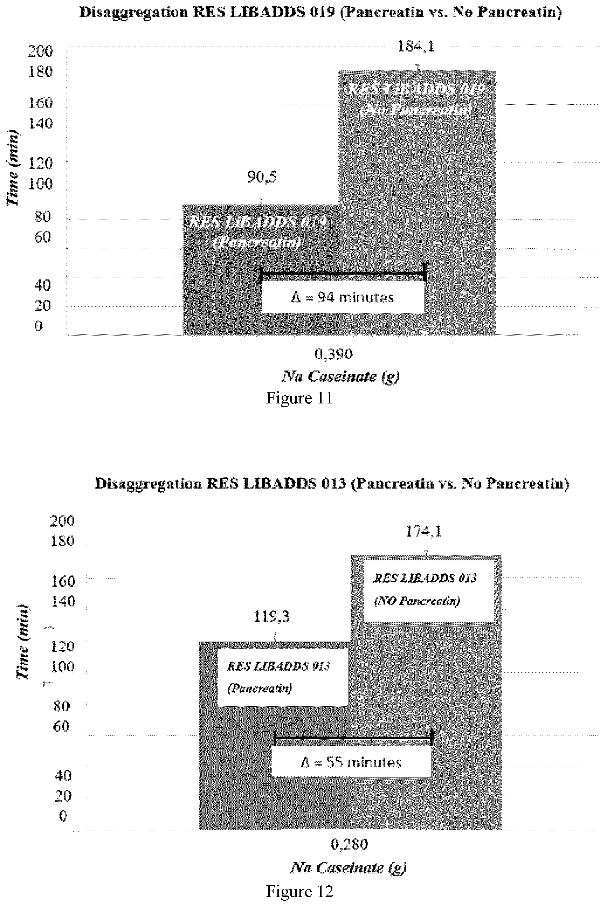

Regarding tablets containing NaC, the presence of pancreatin plays a central role in promoting tablet disaggregation, confirming that NaC functions as a "modulating release matrix" (Figure 10-12).

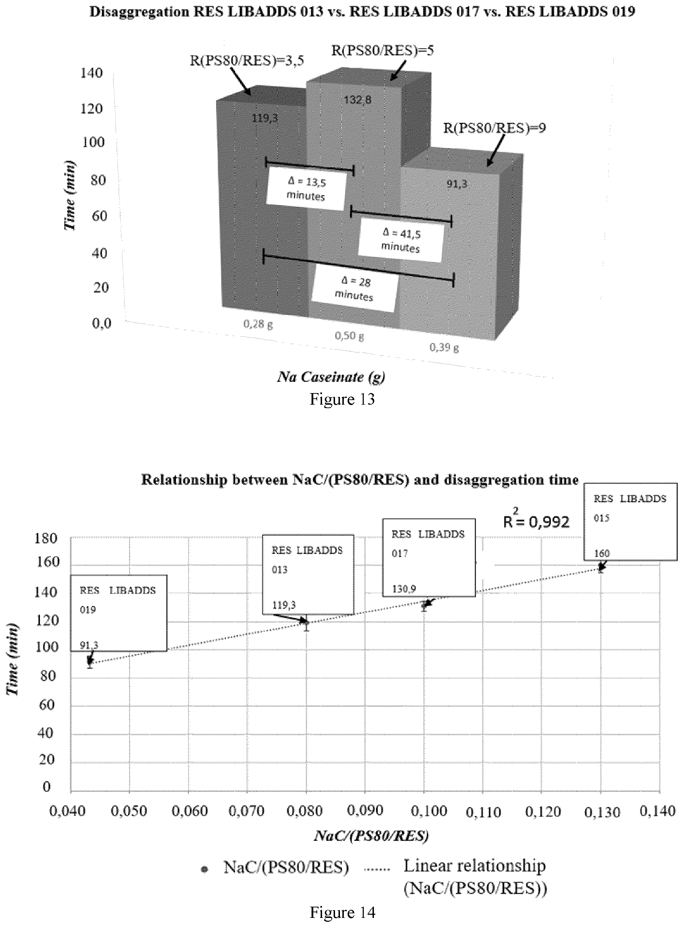

The lowest disaggregation time was measured with RES LiBADDS 019, which contains the highest PS80/RES ratio and the lowest NaC/(PS80/RES) ratio (Figures 9 and 13).

To further confirm these data, the largest difference in disaggregation time between NaC-containing tablets and LiBADDS-containing tablets with the same NaC content (101 minutes) was recorded for RES LiBADDS 019 (Figures 9 and 13).

From the collected data, a pseudo-linear correlation between the NaC/(PS80/RES) ratio and the disaggregation time can be established, according to Figure 14. This correlation suggests that the higher the NaC and LiBADDS content with the same NaC content (101 minutes). This correlation suggests, again, that the higher the PS80/RES ratio, and the lower the NaC content, the faster the breakdown of RES LiBADDS tablets.

A probable explanation for this phenomenon could lie in the optimized solubilization of the RES in the LiBADDS phase containing the highest PS80/RES ratio. Therefore, it can be stated that the solubilization rate of the RES in the LiBADDS step is closely related to the PS80 content and directly related to the disaggregation time.

### 5.2.5. Bioaccessible fraction of RES in mixed micelles in FaSSIF

Table 3. Bioaccessible fraction of RES in mixed micelles generated by the dispersion of RES LiBADDS 013, 015, 017, 019, not formulated in FaSSIF.

| | RES (*cis+trans*) FaSSIF pellets (6 tablets) mg | FaSSIF supernatant (*cis+trans*) RES (6 tablets) mg | RES (*cis+trans*) recovery (6 tablets) mg | RES (*cis+trans*) theoretical (6 tablets) mg | Bioaccessible Fraction (BF) (%) |
|---|---|---|---|---|---|
| **RES LiBADDS 013** | 1.76 ± 0.14 | 36.55 ± 3.8 | 49.8 ± 3.6 | 60 | 73.4 |

(continued)

| | RES (*cis+trans*) FaSSIF pellets (6 tablets) mg | FaSSIF supernatant (*cis+trans*) RES (6 tablets) mg | RES (*cis+trans*) recovery (6 tablets) mg | RES (*cis+trans*) theoretical (6 tablets) mg | Bioaccessible Fraction (BF) (%) |
|---|---|---|---|---|---|
| **RES LiBADDS 015** | 6.40 ± 0.52 | 43.6 ± 4.6 | 54.9 ± 5.8 | 60 | 79.4 |
| **RES LiBADDS 017** | 4.80 ± 0.26 | 44.4 ± 3.5 | 51.0 ± 6.8 | 60 | 87.0 |
| **RES LiBADDS 019** | 1.20 ± 0.19 | 24.8 ± 1.8 | 26.4 ± 3.9 | 30 | 94.0 |
| **Unformulated RES** | 0 | 29.6 ± 1.4 | 29.6 ± 3.9 | 30 | 98.7 |

$$(1) \qquad BF = (RES_{mic}/ RES_{tot})*100$$

wherein: BF is the bioaccessible fraction, $RES_{mic}$ is the fraction of RES encapsulated in the supernatant mixed micelle and $RES_{tot}$ is the total RES loaded into the tablets recovered from the HPLC-DAD analysis.

According to equation (1), the BF of RES in the mixed micelle generated after the dispersion of RES LiBADDS 013, 015, 017, 019 and not formulated in FaSSIF, is respectively 76%, 79%, 87%, 94, 99%. These values mean that RES are usefully trapped in micelles representing the bioaccessible fraction.

In contrast, the dislocated RES in the pellet, the non-bioaccessible fraction, is about 3.5%, 11.6%, 9.4%, 4.5%, and 0.

Among others, LiBADDS RES 019 seems to generate the highest bioaccessible fraction, although it should be noted that the unformulated RES shows the highest EE without pellets collected after centrifugation, in accordance with the evidence of a very high bioaccessibility of the RES in the intestine described by Walle et Maier/Salamon [9,20]. In fact, RES is a substance belonging to the BSC II class, therefore with poor solubility in water, but high intestinal permeability.

From these data it can be argued that the higher the PS80/RES ratio, the higher the bioaccessible fraction, in agreement with the hypothesis that the solubilization of RES represents a crucial factor for the improvement of bioaccessibility.

Therefore, the experiments conducted have shown that the RES, even without LiBADDS technology, therefore physiologically, shows good bioaccessibility, i.e. good solubility in the aqueous fraction of the digest, but then undergoes massive biotransformation in the enterocyte and liver microsomes. LiBADDS, on the other hand, manages to guarantee the *same bioaccessibility* of the RES, but is able to break down the biotransformation in the enterocyte, hence the data on the increased permeability in the Caco-2 monolayer of the LiBADDS RES compared to the unformulated RES.

The LiBADDS technology presented here has been shown to significantly increase the permeation of the RES through the Caco-2 monolayer *in vitro* compared to the unformulated RES, thus demonstrating an active role of the LiBADDS matrix in reducing pre-systemic intestinal metabolism and reducing *ATP-binding cassette* activity.

### 5.2.6. Dissolution test

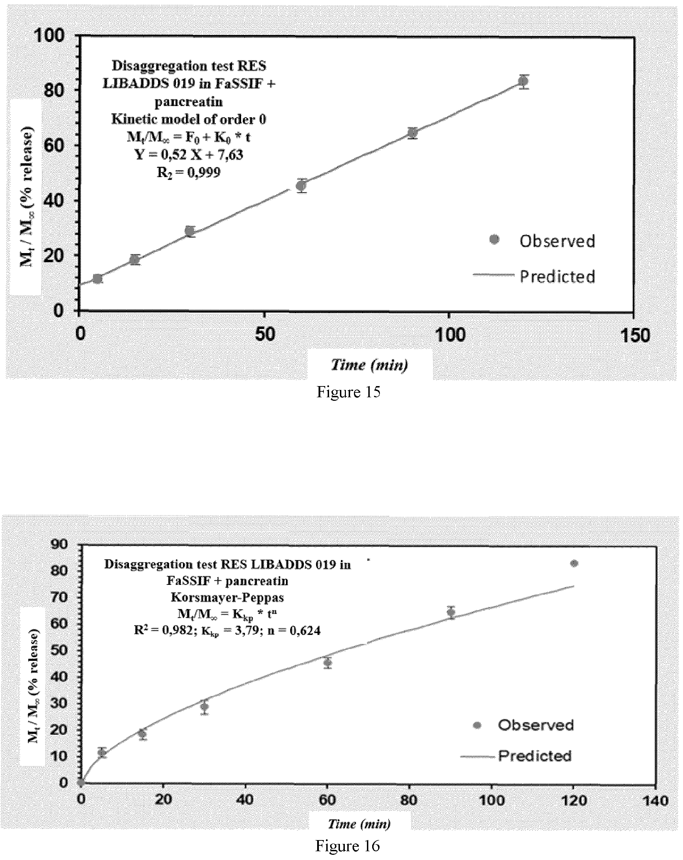

The pseudo-linear trend ($R^2$=0.999) of the graph plotting RES concentration versus time (Figure 15, Table 4) clearly suggests zero-order administration kinetics with a minimum initial *burst,* according to the zero-order equation,

$$(3) \ M_t/M_\infty = M_0 + K_t$$

wherein $M_t$ is the fractional drug release at time t, $M_\infty$ is the amount of drug loaded at infinite time, $M_0$ is the fraction of drug released at t=0; K is a kinetic constant that measures the release rate.

On the other hand, the Korsmayer-Peppas model (3) [21,22] appears to be less suitable for describing the release profile than the zero-order model ($R^2$=0.982, $K_{kp}$ = 3.79, *n = 0.624; DDSolver 1.0 add-on Microsoft Excel software*) (Figure 16, Table 5), but still indicates a probable non-fickian transport of the drug through the matrix and a prevalent release erosion mechanism, according to Table 5.

$$(4) \ M_t/M_\infty = K_{kp} * t^n$$

wherein $M_t$ is the fractional drug release at time t, $M_\infty$ is the amount of drug loaded at infinite time; $K_{kp}$ is the Korsmayer-

Peppas constant that considers the structural and geometric characteristics of the delivery system, and *n* is the parameter that describes the mechanism of drug transport through the matrix.

Table 4. Main parameters of the LiBADDS 019 RES profile graph according to the zero order kinetic models and Korsmayer/Peppas.

|  | Zero-order kinetics | Korsmayer/Peppas kinetics |
|---|---|---|
| Equation | $M_t/M_\infty = M_0 + Kt$ | $M_t/M_\infty = K_{kp} * t^n$ |
| $R^2$ | 0.999 | 0.982 |
| K | 0.52 | - |
| $K_{kp}$ | - | 3.79 |
| *n* | - | 0.62 |

Table 5. Correlation between the transport and release mechanisms and the *nth* exponent of the Korsmayer-Peppas model.

| Release exponent (*n*) | Drug-transporting mechanism | Speed as a function of time | Drug release mechanism |
|---|---|---|---|
| (n = 0.5) | Quasi-Fickian Spreading | $t^n$ | Matrix not inflatable |
| (n = 0.5) | Fickian Spreading | $t^{0.5}$ | Matrix not inflatable |
| 0,5 < *n* < 1 | Abnormal (Non-Fickian transport) | $t^{n-1}$ | Erosion |
| (n = 1) | Transport Case II | (independent time) | Zero order |
| *n* > 1 | Transport Super Case II | $t^{n-1}$ | Relaxation-erosion |

The above data suggest that LiBADDS technology favors a constant release of RES over time, through a slow process of imbibition and erosion of the tablet in the FaSSIF medium.

It is believed that the NaC-based matrix is gradually hydrolyzed by the proteolytic enzymes of pancreatin, resulting in a regular release of RES, independent of the relative concentration. This behavior seems to confirm the "slow-release matrix" characteristics postulated for the LiBADDS technology.

Interestingly, the total time to complete dissolution of the LiBADDS 019 RES was > 240 minutes, while the total time to breakdown was 90 minutes. These data confirm, once again, the substantial difference of these two tests to predict the administration of a poorly soluble drug from a solid form, according to the EP and ICH guidelines [23,24].

Overall, LiBADDS RES tablets exhibit the unique characteristics of a matrix composed of a slowly degraded and eroded water-soluble polymer, both through solubilization and cleavage of NaC by proteolytic enzymes. Most likely the solubilization of NaC increases with hydrolysis.

Another key characteristic that can drive the dissolution rate is the presence of different surfactants that function as "water-attracting agents".

Among others, PS80 plays the crucial role of accelerating disaggregation and solubilizing RES.

***REFERENCES***

1. Li-Man Hung, Jan-Kan Chen, Shiang-Suo Huang, Ren-Shen Lee, Ming-Jai Su, Cardioprotective effect of resveratrol, a natural antioxidant derived from grapes, Cardiovascular Research, Volume 47, Issue 3, August 2000, Pages 549-555, https://doi.org/10.1016/S0008-6363(00)00102-4.

2. Wu JM, Hsieh TC. Resveratrol: a cardioprotective substance. Ann N Y Acad Sci. 2011 Jan;1215:16-21. https://doi: 10.1111/j.1749-6632.2010.05854.x.

3. Bertelli AA, Das DK. Grapes, wines, resveratrol, and heart health. J Cardiovasc Pharmacol. 2009 Dec;54(6):468-76. https://doi: 10.1097/FJC.0b013e3181bfaff3.

4. Wong RH, Thaung Zaw JJ, Xian CJ, Howe PR. Regular Supplementation With Resveratrol Improves Bone Mineral Density in Postmenopausal Women: A Randomized, Placebo-Controlled Trial. J Bone Miner Res. 2020 Nov;35(11):2121-2131. https://doi: 10.1097/FJC.0b013e3181bfaff3. https://doi: 10.1002/jbmr.4115.

5. Thaung Zaw JJ, Howe PR, Wong RH. Long-term effects of resveratrol on cognition, cerebrovascular function and cardio-metabolic markers in postmenopausal women: A 24-month randomised, double-blind, placebo-controlled, crossover study. Clin Nutr. 2021 Mar;40(3):820-829. https://doi: 10.1097/FJC.0b013e3181bfaff3. doi: 10.1016/j.clnu.2020.08.025.

6. Miki H, Uehara N, Kimura A, Sasaki T, Yuri T, Yoshizawa K, Tsubura A. Resveratrol induces apoptosis via ROS-triggered autophagy in human colon cancer cells. Int J Oncol. 2012 Apr;40(4):1020-8. https://doi: 10.3892/ijo.2012.1325.

7. Gertz M, Nguyen GT, Fischer F, Suenkel B, Schlicker C, Fränzel B, Tomaschewski J, Aladini F, Becker C, Wolters D, Steegborn C. A molecular mechanism for direct sirtuin activation by resveratrol. PLoS One. 2012;7(11):e49761. https://doi: 10.1371/journal.pone.0049761.

8. Walle T. Bioavailability of resveratrol. Ann N Y Acad Sci. 2011; 1215:9-15. https://doi: 10.1111/j.1749-6632.2010.05842.x.

9. Walle T, Hsieh F, DeLegge MH, Oatis JE Jr, Walle UK. High absorption but very low bioavailability of oral resveratrol in humans. Drug Metab Dispos. 2004 Dec;32(12):1377-82. https://doi: 10.1124/dmd.104.000885.

10. Francioso A, Mastromarino P, Masci A, d'Erme M, Mosca L. Chemistry, stability and bioavailability of resveratrol. Med Chem. 2014 May;10(3):237-45. https://doi: 10.2174/15734064113096660053.

11. Vitaglione P, Sforza S, Galaverna G, Ghidini C, Caporaso N, Vescovi PP, Fogliano V, Marchelli R. Bioavailability of trans-resveratrol from red wine in humans. Mol Nutr Food Res. 2005 May;49(5):495-504. https://doi: 10.1002/mnfr.200500002.

12. Vesely O, Baldovska S, Kolesarova A. Enhancing Bioavailability of Nutraceutically Used Resveratrol and Other Stilbenoids. Nutrients. 2021 Sep 2;13(9):3095. https://doi: 10.3390/nu13093095. PMID: 34578972.

13. Zhu Y, Ye J, Zhang Q. Self-emulsifying Drug Delivery System Improve Oral Bioavailability: Role of Excipients and Physico-chemical Characterization. Pharm Nanotechnol. 2020;8(4):290-301. doi: 10.2174/2211738508666200811104240.

14. Rani ER, Radha GV. Insights into Novel Excipients of Self-Emulsifying Drug Delivery Systems and Their Significance: An Updated Review. Crit Rev Ther Drug Carrier Syst. 2021;38(2):27-74. doi: 10.1615/CritRevTherDrugCarrierSyst.2020034975.

15. Salawi A. Self-emulsifying drug delivery systems: a novel approach to deliver drugs. Drug Deliv. 2022 Dec;29(1):1811-1823. doi: 10.1080/10717544.2022.2083724.

16. Pandey V, Kohli S. Lipids and Surfactants: The Inside Story of Lipid-Based Drug Delivery Systems. Crit Rev Ther Drug Carrier Syst. 2018;35(2):99-155. doi: 10.1615/CritRevTherDrugCarrierSyst.2018016710.

17. Cerpnjak K, Zvonar A, Gašperlin M, Vrečer F. Lipid-based systems as a promising approach for enhancing the bioavailability of poorly water-soluble drugs. Acta Pharm. 2013 Dec;63(4):427-45. doi: 10.2478/acph-2013-0040.

18. Van Slyke, L. L., and A. W. Bosworth. 1913. Valency of molecules and molecular weights of casein and paracasein. J. Biol. Chem. 14:227-230.

19. Wang X, Ye A, Lin Q, Han J, Singh H. Gastric digestion of milk protein ingredients: Study using an in vitro dynamic model. J Dairy Sci. 2018 Aug;101(8):6842-6852. doi: 10.3168/jds.2017-14284.

20. Maier-Salamon A, Hagenauer B, Wirth M, Gabor F, Szekeres T, Jäger W. Increased transport of resveratrol across monolayers of the human intestinal Caco-2 cells is mediated by inhibition and saturation of metabolites. Pharm Res. 2006 Sep;23(9):2107-15. https://doi: 10.1007/s11095-006-9060-z.

21. R.W. Korsmeyer, N.A. Peppas, "Solute and penetrant diffusion in swellable polymers. III. Drug release from glassy poly(HEMA-co-NVP) copolymers", J. Cont. rel. 1(2): 89-98 (1984). https://doi.org/10.1016/0168-3659(84)90001-4.

22. N.A. Peppas, J.J. Sahlin, "A simple equation for the description of solute release. III. Coupling of diffusion and relaxation", Int. J. Pharm. 57: 169-172 (1989). https://doi.org/10.1016/0378-5173(89)90306-2.

23. Radwan, A.; Wagner, M.; Amidon, G. L.; Langguth, P. Biopredictive tablet disintegration: effect of water diffusivity, fluid flow, food composition and test conditions. Eur. J. Pharm. Sci. 2014, 57, 273-279. https://doi: 10.1016/j.ejps.2013.08.03833.

24. Nickerson, B.; Kong, A.; Gerst, P.; Kao, S. Correlation of dissolution and disintegration results for an immediate-release tablet. J.Pharm. Biomed. Anal. 2018, 150, 333-340. https://doi: 10.1016/j.jpba.2017.12.017.

**Claims**

1. A gradual enteric-release oral solid composition comprising

    i) at least one lipophilic active ingredient belonging to BCS class II and/or IV according to the biopharmaceutical classification system, **characterised by** low solubility and high permeability, or low solubility and low permeability,
    ii) a self-emulsifying vehicle comprising or consisting of

        ii-a) a solubilizing oily matrix comprising or consisting of

            - an ester of polyoxyethylene sorbitan with a fatty acid, the fatty acid being saturated or unsaturated and having a number of carbon atoms comprised between 16 and 22,
            - a mono and diglyceride of a fatty acid, the fatty acid being saturated or unsaturated and having a number of carbon atoms comprised between 16 and 22,
            - at least one unsaturated fatty acid having a carbon number comprised between 16 and 20,

        ii-b) a caseinate derivative selected from the group consisting of: sodium caseinate, potassium caseinate, calcium caseinate, and combinations thereof,

    iii) suitable excipients and/or diluents,

        wherein the ratio of formula (I) between the amount of caseinate derivative and the ratio between the amount of the at least one polyoxyethylene sorbitan ester and the amount of the at least one lipophilic active ingredient

$$(I) \quad \frac{amount\ of\ caseinate\ derivative}{\left(\frac{amount\ of\ the\ at\ least\ one\ polyoxyethylene\ sorbitan\ ester}{amount\ of\ the\ at\ least\ one\ lipophilic\ active\ ingredient}\right)}$$

        is comprised between 0.03 and 0.6.

2. Solid composition according to claim 1, wherein the at least one lipophilic active ingredient has a molecular weight comprised between 100 and 1000 g/mol.

3. Solid composition according to claim 1 or 2, wherein the oily matrix is in an amount comprised between 2% and 15% by weight of the total weight of the composition.

4. Solid composition according to any one of claims from 1 to 3, wherein the polyoxyethylene sorbitan ester is selected from the group consisting of: polysorbate 20, polysorbate 40, polysorbate 60, polysorbate 80, and combinations of the foregoing.

5. Solid composition according to any one of claims from 1 to 4, containing resveratrol and/or boswellia and/or bromelain and/or a fat-soluble vitamin and/or a fat-soluble active ingredient soluble in the oily matrix.

6. Solid composition according to any one of claims from 1 to 5, in powder, granulate, tablet, capsule form, preferably in tablet form.

7. Process for preparing the solid composition according to any one of claims from 1 to 6, comprising the following steps:

a) preparing: the at least one active lipophilic ingredient; the polyoxyethylene sorbitan ester with a fatty acid; the mono and diglyceride of a fatty acid; the at least one unsaturated fatty acid; the caseinate derivative; suitable excipients and/or diluents;

b) dissolving or adsorbing the at least one active lipophilic ingredient in the oily matrix comprising the polyoxyethylene sorbitan ester, the mono and diglyceride of a fatty acid, the at least one unsaturated fatty acid, at a temperature comprised between 40°C and 100°C, to obtain a melted oily mixture;

c) adsorbing the melted oily mixture of step (b) above on the caseinate derivative, to obtain the vehicle in the form of a wet powder;

d) adding the vehicle and the suitable excipients and/or diluents.

8. Solid composition according to claim 5, for use in the prevention and/or treatment of a disorder or a disease selected from the group consisting of: osteopenia, osteoporosis, cardiovascular disease, hypertension, venous insufficiency, cancer; disorders that occur in conjunction with or are caused by menopause, preferably disorders such as hot flushes and peripheral vascular disorders, and/or for chrono and photo-ageing of the skin; and combinations of the foregoing.

Figure 1

(A)                                    (B)

Figure 2

**Calibration curve_RESVERATROL 98%**

$y = 46,894x - 59,002$
$R^2 = 0,9997$

Figure 3

NaC in FSSGF (NO pepsin) (sx) | NaC in FSSGF/pepsin (dx)

RES-LIBADDS in FSSGF (NO pepsin) (sx) | RES-LIBADDS in FSSGF/pepsin (dx)

Figure 4

Figure 5

Figure 6

**Disaggregation RES LIBADDS 013 vs. Na Caseinate 0.28g**

Figure 7

**Disaggregation RES LIBADDS 017 vs. Na Caseinate 0.500g**

Figure 8

**Disaggregation RES LIBADDS 019 vs. Na Caseinate 0.39g**

Figure 9

**Disaggregation RES LIBADDS 017 (Pancreatin vs. No Pancreatin)**

Figure 10

**Disaggregation RES LIBADDS 019 (Pancreatin vs. No Pancreatin)**

Figure 11

**Disaggregation RES LIBADDS 013 (Pancreatin vs. No Pancreatin)**

Figure 12

EP 4 678 167 A1

**Disaggregation RES LIBADDS 013 vs. RES LIBADDS 017 vs. RES LIBADDS 019**

Figure 13

**Relationship between NaC/(PS80/RES) and disaggregation time**

Figure 14

Figure 15

Figure 16

Europäisches
Patentamt
European
Patent Office
Office européen
des brevets

## EUROPEAN SEARCH REPORT

**Application Number**

EP 25 18 5883

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/193380 A2 (SOLURAL PHARMA APS [DK]) 23 December 2015 (2015-12-23) * page 4, line 9 - line 31 * * page 18, line 21 - page 24, line 13 * * examples 1-7 * | 1-8 | INV. A61K9/20 A61K31/05 A61P9/12 A61P15/12 A61P19/10 |
| A | MORA-GUTIERREZ ADELA ET AL: "Interface Compositions as Determinants of Resveratrol Stability in Nanoemulsion Delivery Systems", FOODS, vol. 9, no. 10, 2 October 2020 (2020-10-02), page 1394, XP093257291, CH ISSN: 2304-8158, DOI: 10.3390/foods9101394 * the whole document * | 1-8 | A61P35/00 |
| A | WO 2017/215791 A1 (AQUANOVA AG [DE]) 21 December 2017 (2017-12-21) * the whole document * | 1-8 | |
| A | CN 117 502 516 A (BEIJING ACAD AGRIC & FORESTRY) 6 February 2024 (2024-02-06) * the whole document * | 1-8 | TECHNICAL FIELDS SEARCHED (IPC) A61K A61P |
| A | US 2012/225118 A1 (CHEN HAILIANG [US] ET AL) 6 September 2012 (2012-09-06) * paragraph [0004] * * paragraph [0021] - paragraph [0026] * * paragraph [0044] * * paragraph [0059] - paragraph [0075] * * examples 4-6 * | 1-8 | |
| A | WO 94/03158 A1 (MERZ & CO GMBH & CO [DE]) 17 February 1994 (1994-02-17) * the whole document * | 1-8 | |

The present search report has been drawn up for all claims

| Place of search | Date of completion of the search | Examiner |
|---|---|---|
| Munich | 26 November 2025 | Giró, Annalisa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 18 5883

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2025

| Patent document cited in search report | Publication date | Patent family member(s) | Publication date |
|---|---|---|---|
| WO 2015193380 A2 | 23-12-2015 | AU 2015276202 A1 | 02-02-2017 |
| | | BR 112016029271 A2 | 22-08-2017 |
| | | CA 2948225 A1 | 23-12-2015 |
| | | CN 107073127 A | 18-08-2017 |
| | | CN 115463106 A | 13-12-2022 |
| | | DK 3157508 T3 | 15-02-2021 |
| | | EA 201790027 A1 | 31-05-2017 |
| | | EP 3157508 A2 | 26-04-2017 |
| | | ES 2851332 T3 | 06-09-2021 |
| | | HU E054467 T2 | 28-09-2021 |
| | | JP 6723166 B2 | 15-07-2020 |
| | | JP 7071420 B2 | 18-05-2022 |
| | | JP 2017518294 A | 06-07-2017 |
| | | JP 2020090538 A | 11-06-2020 |
| | | KR 20170020479 A | 22-02-2017 |
| | | MX 377352 B | 07-03-2025 |
| | | NZ 728110 A | 29-09-2023 |
| | | PL 3157508 T3 | 17-05-2021 |
| | | PT 3157508 T | 17-02-2021 |
| | | SG 11201609352T A | 27-01-2017 |
| | | US 2017119674 A1 | 04-05-2017 |
| | | WO 2015193380 A2 | 23-12-2015 |
| | | ZA 201700301 B | 26-01-2022 |
| WO 2017215791 A1 | 21-12-2017 | CA 3026810 A1 | 21-12-2017 |
| | | CN 109562066 A | 02-04-2019 |
| | | DK 3468535 T3 | 06-07-2020 |
| | | EP 3468535 A1 | 17-04-2019 |
| | | ES 2798403 T3 | 11-12-2020 |
| | | HU E049304 T2 | 28-09-2020 |
| | | JP 6907243 B2 | 21-07-2021 |
| | | JP 2019518047 A | 27-06-2019 |
| | | PL 3468535 T3 | 07-09-2020 |
| | | US 2019307700 A1 | 10-10-2019 |
| | | WO 2017215791 A1 | 21-12-2017 |
| CN 117502516 A | 06-02-2024 | NONE | |
| US 2012225118 A1 | 06-09-2012 | EP 2470019 A1 | 04-07-2012 |
| | | JP 2013503166 A | 31-01-2013 |
| | | US 2012225118 A1 | 06-09-2012 |
| | | US 2013079334 A1 | 28-03-2013 |
| | | WO 2011025771 A1 | 03-03-2011 |
| | | WO 2011031462 A2 | 17-03-2011 |
| WO 9403158 A1 | 17-02-1994 | AT E176866 T1 | 15-03-1999 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 25 18 5883

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2025

| Patent document cited in search report | Publication date | Patent family member(s) | | Publication date |
|---|---|---|---|---|
| | | AU | 669731 B2 | 20-06-1996 |
| | | CA | 2141691 A1 | 17-02-1994 |
| | | CN | 1086708 A | 18-05-1994 |
| | | DE | 4225730 A1 | 10-02-1994 |
| | | DK | 0582186 T3 | 27-09-1999 |
| | | EP | 0582186 A1 | 09-02-1994 |
| | | ES | 2128369 T3 | 16-05-1999 |
| | | GR | 3030227 T3 | 31-08-1999 |
| | | IL | 106580 A | 08-02-1998 |
| | | JP | 3560244 B2 | 02-09-2004 |
| | | JP | H07509479 A | 19-10-1995 |
| | | LT | 3201 B | 27-03-1995 |
| | | LV | 10182 A | 20-10-1994 |
| | | US | 5382601 A | 17-01-1995 |
| | | WO | 9403158 A1 | 17-02-1994 |
| | | ZA | 935614 B | 03-02-1995 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GEBOERS S** ; **STAPPAERTS J** ; **TACK J** ; **ANNAERT P** ; **AUGUSTIJNS P**. In vitro and in vivo investigation of the gastrointestinal behavior of simvastatin. *Int J Pharm.*, 20 August 2016, vol. 510 (1), 296-303
- **GEBOERS S** ; **STAPPAERTS J** ; **TACK J** ; **ANNAERT P** ; **AUGUSTIJNS P**. In vitro and in vivo investigation of the gastrointestinal behavior of simvastatin.. *Int J Pharm.*, 20 August 2016, vol. 510 (1), 296-303
- **LI-MAN HUNG** ; **JAN-KAN CHEN** ; **SHIANG-SUO HUANG** ; **REN-SHEN LEE** ; **MING-JAI SU**. Cardioprotective effect of resveratrol, a natural antioxidant derived from grapes. *Cardiovascular Research*, August 2000, vol. 47 (3), 549-555, https://doi.org/10.1016/S0008-6363(00)00102-4
- **WU JM** ; **HSIEH TC**. Resveratrol: a cardioprotective substance. *Ann N Y Acad Sci.*, January 2011, vol. 1215, 16-21, https://doi: 10.1111/j.1749-6632.2010.05854.x
- **BERTELLI AA** ; **DAS DK**. Grapes, wines, resveratrol, and heart health.. *J Cardiovasc Pharmacol.*, December 2009, vol. 54 (6), 468-76, https://doi: 10.1097/FJC.0b013e3181bfaff3
- **WONG RH** ; **THAUNG ZAW JJ** ; **XIAN CJ** ; **HOWE PR**. Regular Supplementation With Resveratrol Improves Bone Mineral Density in Postmenopausal Women: A Randomized, Placebo-Controlled Trial. *J Bone Miner Res.*, November 2020, vol. 35 (11), 2121-2131, https://doi: 10.1097/FJC.0b013e3181bfaff3. https://doi: 10.1002/jbmr.4115
- **THAUNG ZAW JJ** ; **HOWE PR** ; **WONG RH**. Long-term effects of resveratrol on cognition, cerebrovascular function and cardio-metabolic markers in postmenopausal women: A 24-month randomised, double-blind, placebo-controlled, crossover study. *Clin Nutr.*, March 2021, vol. 40 (3), 820-829, https://doi: 10.1097/FJC.0b013e3181bfaff3. doi: 10.1016/j.clnu.2020.08.025
- **MIKI H** ; **UEHARA N** ; **KIMURA A** ; **SASAKI T** ; **YURI T** ; **YOSHIZAWA K** ; **TSUBURA A.** Resveratrol induces apoptosis via ROS-triggered autophagy in human colon cancer cells.. *Int J Oncol*, April 2012, vol. 40 (4), 1020-8, https://doi: 10.3892/ijo.2012.1325

- **GERTZ M** ; **NGUYEN GT** ; **FISCHER F** ; **SUENKEL B** ; **SCHLICKER C** ; **FRÄNZEL B** ; **TOMASCHEWSKI J** ; **ALADINI F** ; **BECKER C** ; **WOLTERS D**. A molecular mechanism for direct sirtuin activation by resveratrol. *PLoS One*, 2012, vol. 7 (11), e49761, https://doi: 10.1371/journal.pone.0049761
- **WALLE T**. Bioavailability of resveratrol. *Ann N Y Acad Sci.*, 2011, vol. 1215, 9-15, https://doi: 10.1111/j.1749-6632.2010.05842.x
- **WALLE T** ; **HSIEH F** ; **DELEGGE MH** ; **OATIS JE JR** ; **WALLE UK**. High absorption but very low bioavailability of oral resveratrol in humans.. *Drug Metab Dispos*, December 2004, vol. 32 (12), 1377-82, https://doi: 10.1124/dmd.104.000885
- **FRANCIOSO A** ; **MASTROMARINO P** ; **MASCI A** ; **D'ERME M** ; **MOSCA L**. Chemistry, stability and bioavailability of resveratrol. *Med Chem.*, May 2014, vol. 10 (3), 237-45, https://doi: 10.2174/15734064113096660053
- **VITAGLIONE P** ; **SFORZA S** ; **GALAVERNA G** ; **GHIDINI C** ; **CAPORASO N** ; **VESCOVI PP** ; **FOGLIANO V** ; **MARCHELLI R.** Bioavailability of trans-resveratrol from red wine in humans. *Mol Nutr Food Res.*, May 2005, vol. 49 (5), 495-504, https://doi: 10.1002/mnfr.200500002
- **VESELY O** ; **BALDOVSKA S** ; **KOLESAROVA A**. Enhancing Bioavailability of Nutraceutically Used Resveratrol and Other Stilbenoids. *Nutrients*, 02 September 2021, vol. 13 (9), 3095, https://doi: 10.3390/nu13093095
- **ZHU Y** ; **YE J** ; **ZHANG Q**. Self-emulsifying Drug Delivery System Improve Oral Bioavailability: Role of Excipients and Physico-chemical Characterization. *Pharm Nanotechnol.*, 2020, vol. 8 (4), 290-301
- **RANI ER** ; **RADHA GV**. Insights into Novel Excipients of Self-Emulsifying Drug Delivery Systems and Their Significance: An Updated Review. *Crit Rev Ther Drug Carrier Syst*, 2021, vol. 38 (2), 27-74
- **SALAWI A**. Self-emulsifying drug delivery systems: a novel approach to deliver drugs.. *Drug Deliv*, December 2022, vol. 29 (1), 1811-1823
- **PANDEY V** ; **KOHLI S**. Lipids and Surfactants: The Inside Story of Lipid-Based Drug Delivery Systems. *Crit Rev Ther Drug Carrier Syst.*, 2018, vol. 35 (2), 99-155

- **CERPNJAK K** ; **ZVONAR A** ; **GAŠPERLIN M** ; **VREČER F**. Lipid-based systems as a promising approach for enhancing the bioavailability of poorly water-soluble drugs. *Acta Pharm.*, December 2013, vol. 63 (4), 427-45
- **VAN SLYKE, L. L** ; **A. W. BOSWORTH**. Valency of molecules and molecular weights of casein and paracasein.. *J. Biol. Chem.*, 1913, vol. 14, 227-230
- **WANG X** ; **YE A** ; **LIN Q** ; **HAN J** ; **SINGH H**. Gastric digestion of milk protein ingredients: Study using an in vitro dynamic model. *J Dairy Sci.*, August 2018, vol. 101 (8), 6842-6852
- **MAIER-SALAMON A** ; **HAGENAUER B** ; **WIRTH M** ; **GABOR F** ; **SZEKERES T** ; **JÄGER W**. Increased transport of resveratrol across monolayers of the human intestinal Caco-2 cells is mediated by inhibition and saturation of metabolites. *Pharm Res.*, September 2006, vol. 23 (9), 2107-15, https://doi: 10.1007/s11095-006-9060-z
- **R.W. KORSMEYER** ; **N.A. PEPPAS**. Solute and penetrant diffusion in swellable polymers. III. Drug release from glassy poly(HEMA-co-NVP) copolymers. *J. Cont. rel.*, 1984, vol. 1 (2), 89-98, https://doi.org/10.1016/0168-3659(84)90001-4
- **N.A. PEPPAS** ; **J.J. SAHLIN**. A simple equation for the description of solute release. III. Coupling of diffusion and relaxation. *Int. J. Pharm.*, 1989, vol. 57, 169-172, https://doi.org/10.1016/0378-5173(89) 90306-2
- **RADWAN, A** ; **WAGNER, M** ; **AMIDON, G. L** ; **LANGGUTH, P**. Biopredictive tablet disintegration: effect of water diffusivity, fluid flow, food composition and test conditions.. *Eur. J. Pharm. Sci.*, 2014, vol. 57, 273-279, https://doi: 10.1016/j.ejps.2013.08.03833
- **NICKERSON, B.** ; **KONG, A** ; **GERST, P** ; **KAO, S**. Correlation of dissolution and disintegration results for an immediate-release tablet. *J.Pharm. Biomed. Anal.*, 2018, vol. 150, 333-340, https://doi: 10.1016/j.jpba.2017.12.017